# EUROPÄISCHE PATENTSCHRIFT

(11) **EP 1 294 682 B1**
(45) Veröffentlichungstag und Bekanntmachung des Hinweises auf die Patenterteilung: **28.04.2004**
(21) Anmeldenummer: 01936399.3
(22) Anmeldetag: 26.05.2001
(51) Int. Cl.: C07C 275/34, C07D 295/08, C07D 307/38, A61K 31/19, A61K 31/215, A61K 31/34, A61P 3/10

(54) **ACYLPHENYLHARNSTOFFDERIVATE, VERFAHREN ZU IHRER HERSTELLUNG UND IHRE VERWENDUNG ALS ARZNEIMITTEL**
ACYLPHENYL UREA DERIVATIVES, METHODS FOR THE PRODUCTION THEREOF AND USE THEREOF AS A MEDICAMENT
DERIVES D'ACYLPHENYLUREE, LEURS PROCEDES DE PREPARATION ET LEUR UTILISATION COMME MEDICAMENTS

(30) Priorität: 09.06.2000 DE 10028175; 04.04.2001 DE 10116768
(43) Veröffentlichungstag der Anmeldung: 26.03.2003
(73) Patentinhaber: Aventis Pharma Deutschland GmbH, 65929 Frankfurt am Main (DE)
(72) Erfinder: DEFOSSA, Elisabeth, 65510 Idstein (DE); KLABUNDE, Thomas, 65929 Frankfurt (DE); BURGER, Hans-Joerg, Morristown, NJ 07960 (US); HERLING, Andreas, 65520 Bad Camberg (DE); BARINGHAUS, Karl-Heinz, 61200 Wölfersheim (DE)
(86) Internationale Anmeldenummer: PCT/EP2001/006030
(87) Internationale Veröffentlichungsnummer: WO 2001/094300

(56) Entgegenhaltungen:
- EP-A- 0 632 019
- US-A- 3 718 660

## Beschreibung

Die Erfindung betrifft Acylphenylharnstoffderivate sowie deren physiologisch verträgliche Salze und physiologisch funktionelle Derivate.

Es sind bereits Acylphenylharnstoffderivate als Insektizide im Stand der Technik beschrieben (EP 0136 745, EP 0 167 197, DE 29 26 480, J. Agric. Food Chem. 1999, 47, 3116-3424).

US-A-3 718 660 beschreibt Arylsulfonylharnstoff Derivate mit blutzuckersenkender Wirkung.

Der Erfindung lag die Aufgabe zugrunde, alternative Verbindungen zur Verfügung zu stellen, die eine therapeutisch verwertbare Blutzucker senkende Wirkung entfalten.

Die Erfindung betrifft daher Verbindungen der Formel I, 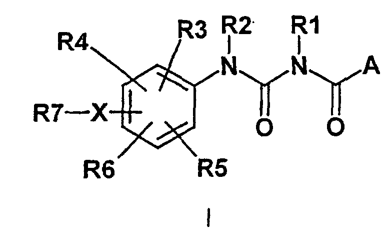 worin bedeuten
- A: Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO(C₁-C₇)-alkyl, CONH₂, CONH(C₁-C₆)-Alkyl, CON[(C₁-C₆)-Alkyl]₂, CONH(C₃-C₆)-Cycloalkyl, (C₀-C₆)-Alkylen-NH₂, (C₀-C₆)-Alkylen-NH(C₂-C₆)-alkyl, (C₀-C₆)-Alkylen-N[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- R1, R2: unabhängig voneinander H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COOH, (C₁-C₆)-Alkylen-COO-(C₁-C₆)-alkyl;
- R3, R4, R5, R6: unabhängig voneinander H, F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, COOH, COO(C₁-C₆)-Alkyl, CONH₂, CONH(C₁-C₆)-Alkyl, CON[(C₁-C₆)-Alkyl]₂, CONH(C₃-C₇)-Cycloalkyl, NH₂, NH(C₁-C₆)-Alkyl, N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- X: O, S;
- R7: (C₁-C₁₀)-Alkylen-COOH, (C₆-C₁₀)-Alkylen-COO-(C₁-C₆)-Alkyl, (C₁-C₁₀)-Alkylen-CONH₂, (C₁-C₁₀)-Alkylen-CONH-(C₁-C₆)-alkyl, (C₁-C₁₀)-Alkylen-CON-[(C₁-C₆)-alkyl]₂, (C₁-C₁₀)-Alkylen-NH₂, (C₁-C₁₀)-Alkylen-NH(C₁-C₆)-Alkyl, (C₁-C₁₀)-Alkylen-N[(C₁-C₆)-Alkyl]₂, (C₁-C₁₀)-Alkylen-B;
- B: (C₃-C₇)-Cycloalkyl, Pyrrolyl, Imidazolyl, Thiazolyl, Azetidinyl, Thienyl, Piperidinyl, Pyrrolidinyl, Morpholinyl, Pyridyl-methyl oder Furyl, worin Cycloalkyl, Pyrrolyl, Imidazolyl, Thiazolyl, Azetidinyl, Thienyl, Piperidinyl, Pyrrolidinyl, Morpholinyl, Pyridyl oder Furyl jeweils bis zu zweimal mit Cl, F, CN, CF₃, OCF₃, COOH, COO-(C₁-C₆)-Alkyl, CONH₂, CONH-(C₁-C₆)-Alkyl, CON-[(C₁-C₆)-Alkyl]₂, (C₁-C₆)-Alkyl, OH, O-(C₁-C₆)-Alkyl substituiert sein können;
sowie deren physiologisch verträgliche Salze,
wobei die Verbindung der Formel 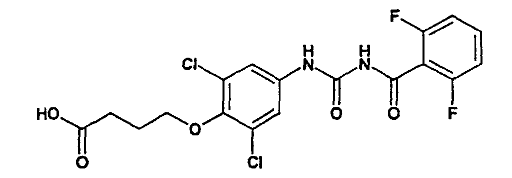 sowie Verbindungen der Formel I, worin die Reste gleichzeitig bedeuten
- A: Phenyl;
- X: O;
- R1: H;
- R7: -(C₁-C₄)-Alkyl-B;
- B: (C₃-C₇)-Cycloalkyl, Heteroaryl;
ausgenommen sind.

Besonders bevorzugt sind Verbindungen der Formel I, worin bedeuten
- A: Phenyl, wobei der Phenylrest bis zu zweifach substituiert sein kann mit F, Cl, Br, O-(C₁-C₆)-Alkyl;
- R1, R2: unabhängig voneinander H, (C₁-C₆)-Alkyl, CO-(C₁-C₆)-Alkyl;
- R3, R4, R5, R6: unabhängig voneinander H, Cl, F, (C₁-C₆)-Alkyl, O-(C₁-C₆)-Alkyl, -COO-(C₁-C₆)-Alkyl;
- X: O;
- R7: (C₁-C₁₀)-Alkylen-COOH, (C₆-C₁₀)-Alkylen-COO-(C₁-C₆)-Alkyl, (C₁-C₁₀)-Alkylen-CONH₂;
sowie deren physiologisch verträgliche Salze,
wobei die Verbindung der Formel 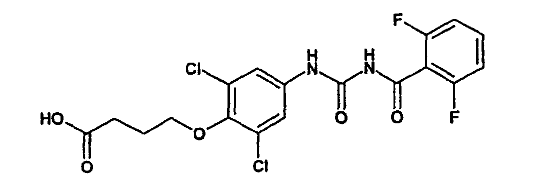 ausgenommen ist.

Die Erfindung bezieht sich weiterhin auf die Verwendung der Verbindungen der Formel 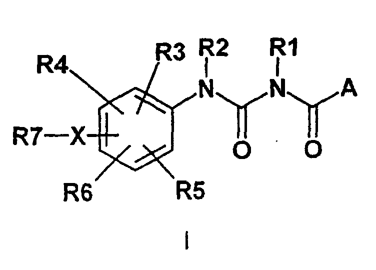 worin bedeuten
- A: Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO(C₁-C₆)-alkyl, CONH₂, CONH(C₁-C₆)-Alkyl, CON[(C₁-C₆)-Alkyl]₂, CONH(C₃-C₇)-Cycloalkyl, (C₀-C₆)-Alkylen-NH₂, (C₀-C₆)-Alkylen-NH(C₁-C₆)-Alkyl, (C₀-C₆)-Alkylen-N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- R1, R2: unabhängig voneinander H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COOH, (C₁-C₆)-Alkylen-COO-(C₁-C₆)-alkyl;
- R3, R4, R5,: unabhängig voneinander H, F, Cl, Br, OH, CF₃, NO₂, CN, OCF₃, O-(C₁-C₆)-Alkyl, O-(C₁=C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, COOH, COO(C₁-C₆)-Alkyl, CONH₂, CONH(C₁-C₆)-Alkyl, CON[(C₁-C₆)-Alkyl]₂, CONH(C₃-C₇)-Cycloalkyl, NH₂, NH(C₁-C₆)-Alkyl, N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
- X: O, S;
- R7: (C₁-C₁₀)-Alkylen-COOH, (C₁-C₁₀)-Alkylen-COO-(C₁-C₆)-alkyl, (C₁-C₁₀)-Alkylen-CONH₂, (C₁-C₁₀)-Alkylen-CONH-(C₁-C₆)-alkyl, (C₁-C₁₀)-Alkylen-CON-[(C₁-C₆)-alkyl]₂, (C₁-C₁₀)-Alkylen-NH₂, (C₁-C₁₀)-Alkylen-NH(C₁-C₆)-alkyl, (C₁-C₁₀)-Alkylen-N[(C₁-C₆)-alkyl]₂, (C₁-C₁₀)-Alkylen-B;
- B: (C₃-C₇)-Cycloalkyl, Phenyl, Pyrrolyl, Imidazolyl, Thiazolyl, Azetidinyl, Thienyl, Piperidinyl, Pyrrolidinyl, Morpholinyl, Pyridyl oder Furyl, worin Cycloalkyl, Phenyl, Pyrrolyl, Imidazolyl, Thiazolyl, Azetidinyl, Thienyl, Piperidinyl, Pyrrolidinyl, Morpholinyl, Pyridyl oder Furyl jeweils bis zu zweimal mit Cl, F, CN, CF₃, OCF₃, COOH, COO-(C₁-C₆)-Alkyl, CONH₂, CONH-(C₁-C₆)-Alkyl, CON-[(C₁-C₆)-Alkyl]₂ (C₁-C₆)-Alkyl, OH, O-(C₁-C₆)-Alkyl substituiert sein können;
sowie derer physiologisch verträglichen Salze zur Herstellung eines Medikamentes zur Senkung des Blutzuckerspiegels und Behandlung von Typ II Diabetes.

Die Erfindung bezieht sich auf Verbindungen der Formel I, in Form ihrer Racemate, racemischen Mischungen und reinen Enantiomere sowie auf ihre Diastereomere und Mischungen davon.
Die Alkylreste in den Substituenten R1, R2, R3, R4, R5, R6, R7, A und B können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bemstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I gemäß dem vorliegenden Anspruch 11, dadurch gekennzeichnet, daß man die Verbindungen der Formel I so gewinnt, daß gemäß dem folgenden Reaktionsschema vorgegangen wird:

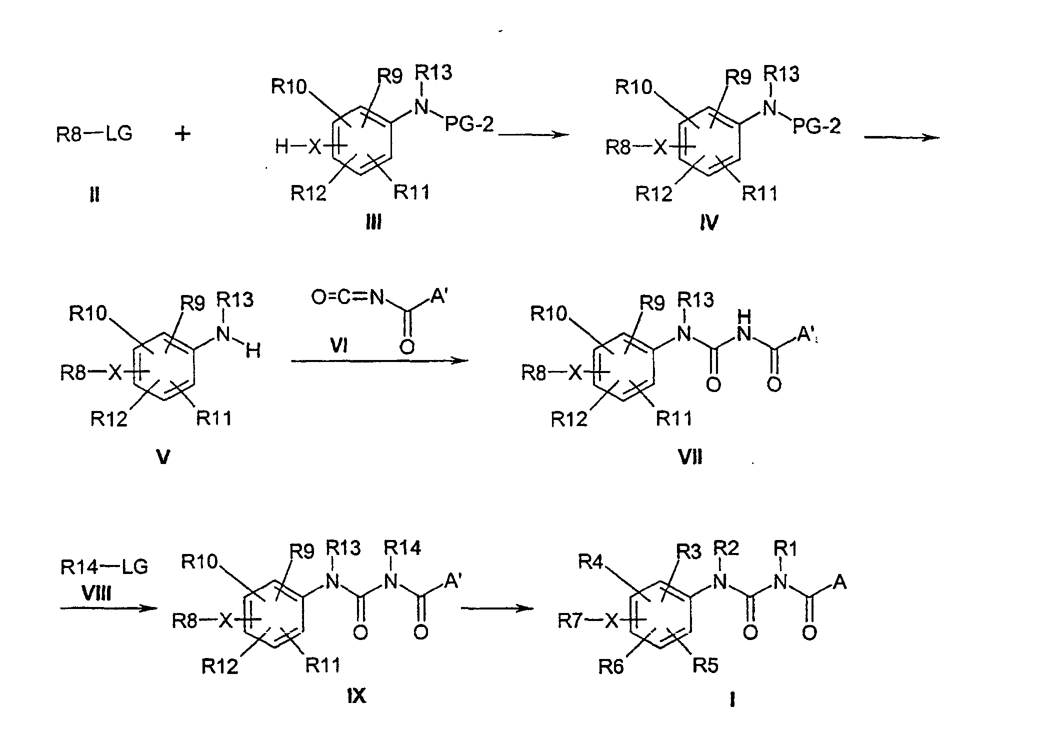

Dazu werden Verbindungen der allgemeinen Formel II,

R8-LG (II)

in denen
- R8: (C₁-C₁₀)-Alkylen-COO-(PG-1), (C₆-C₁₀)-Alkylen-COO-(C₁-C₆)-alkyl, (C₁-C₁₀)-Alkylen-CON-(PG-2)₂, (C₁-C₁₀)-Alkylen-CONH-(C₁-C₆)-alkyl, (C₁-C₁₀)-Alkylen-CON-[(C₁-C₆)-alkyl]₂, (C₁-C₁₀)-Alkylen-N-(PG-2)₂, (C₁-C₁₀)-Alkylen-NH(C₁-C₆)-alkyl, (C₁-C₁₀)-Alkylen-N[(C₁-C₆)-alkyl]₂, (C₁-C₁₀)-Alkylen-B'
darstellt, worin
PG-1 eine allgemein bekannte Schutzgruppe für Ester, wie zum Beispiel (C₁-C₆)-Alkyl, Benzyl oder p-Methoxybenzyl, und
PG-2 eine allgemein bekannte Schutzgruppe für Aminogruppen, wie zum Beispiel (C₁-C₆)-Alkylcarbonyl, (C₁-C₆)-Alkyloxycarbonyl oder (C₆-C₁₂)-Aryl-(C₁-C₄)-alkyloxycarbonyl, die entweder beide Wasserstoffe oder nur ein Wasserstoffatom der Aminogruppe ersetzt, und
- B': (C₃-C₇)-Cycloalkyl, Pyrrolyl, Imidazolyl, Thiazolyl, Azetidinyl, Thienyl, Piperidinyl, Pyrrolidinyl, Morpholinyl, Pyridyl und Furyl, worin Cycloalkyl, Pyrrolyl, Imidazolyl, Thiazolyl, Azetidinyl, Thienyl, Piperidinyl, Pyrrolidinyl, Morpholinyl, Pyridyl und Furyl jeweils bis zu zweimal mit Cl, F, CN, CF₃, OCF₃, COO-(PG-1), COO-(C₁-C₆)-Alkyl, CON-(PG-2)₂, CONH-(C₁-C₆)-Alkyl, CON-[(C₁-C₆)-Alkyl]₂, (C₁-C₆)-Alkyl, O-(PG-3), O-(C₁-C₆)-Alkyl substituiert sein können
worin PG-3 eine allgemein bekannte Schutzgruppe für Alkohole, wie zum Beispiel Benzyl, Allyl, Tetrahydropyranyl oder Tetrahydrofuranyl, darstellt,
und
LG eine allgemein bekannte Austrittsgruppe, wie zum Beispiel Halogen, Arylsulfonyloxy oder Alkylsulfonyloxy, darstellt,
mit Anilinen der allgemeinen Formel III 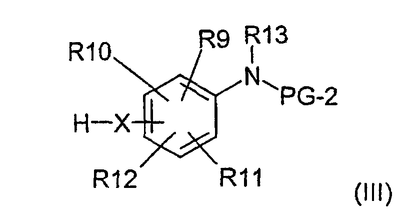 in denen X und PG-2 die oben beschriebene Bedeutung haben und
- R9, R10, R11, R12: unabhängig voneinander H, F, Cl, Br, O-(PG-3), CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-N-(PG-2)₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, COO-(PG-1), COO(C₁-C₆)-Alkyl, CON-(PG-2)₂, CONH(C₁-C₆)-Alkyl, CON[(C₁-C₆)-Alkyl]₂, CONH(C₃-C₇)-Cycloalkyl, N-(PG-2)₂, NH(C₁-C₆)-Alkyl, N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, O-(PG-3), (C₁-C₆)-Alkyl, O-(C₁-C₆)-Alkyl, CF₃, OCF₃, COO-(PG-1), COO(C₁-C₆)-Alkyl oder CON-(PG-2)₂ substituiert sein kann;
- R13: H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COO-(PG-1), (C₁-C₆)-Alkylen-COO-(C₁-C₆)-alkyl darstellt,
wobei PG-1, PG-2 und PG-3 die oben beschriebene Bedeutung haben
unter Verwendung einer Base, wie zum Beispiel Kalium- oder Cäsiumkarbonat, in einem organische Lösungsmittel, wie zum Beispiel Aceton oder Dimethylformamid, zu Verbindungen der allgemeinen Formel IV 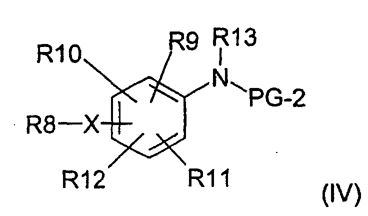 in denen X, R8, R9, R10, R11, R12, R13 und PG-2 die oben beschrieben Bedeutung haben,
alkyliert, die Reaktionszeiten betragen zwischen 2 und 24 Stunden und die Reaktionstemperatur liegt zwischen 10°C und dem Siedepunkt des verwendeten Lösungsmittels,
anschließend werden durch selektive Abspaltung der Schutzgruppe PG-2 Verbindungen der allgemeinen Formel V 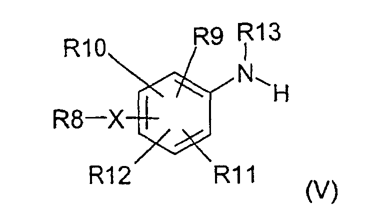 in denen X, R8, R9, R10, R11, R12, und R13 die oben aufgeführten Bedeutungen haben, erhalten,

Verbindungen der allgemeinen Formel V werden mit Isocyanaten der allgemeinen Formel VI 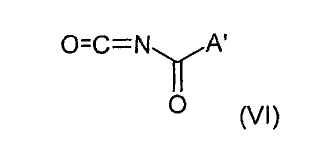 in denen
- A': Phenyl, Naphthyl, wobei der Phenyl- oder Naphthylrest bis zu dreifach substituiert sein kann mit F, Cl, Br, O-(PG-3), CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-N-(PG-2)₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, (C₀-C₆)-Alkylen-COO-(PG-1), (C₀-C₆)-Alkylen-COO(C₁-C₆)-alkyl, CON-(PG-2)₂, CONH(C₁-C₆)-Alkyl, CON[(C₁-C₆)-Alkyl]₂, CONH(C₃-C₇)-Cycloalkyl, (C₀-C₆)-Alkylen-N-(PG-2)₂, (C₀-C₆)-Alkylen-NH(C₁-C₆)-alkyl, (C₀-C₆)-Alkylen-N[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, O-(PG-3), (C₁-C₆)-Alkyl, O-(C₁-C₆)-Alkyl, CF₃, OCF₃, COO-(PG-1), COO(C₁-C₆)-Alkyl oder CON-(PG-2)₂ substituiert sein kann,
wobei PG-1, PG-2 und PG-3 die oben beschriebene Bedeutung haben, darstellt
in wasserfreien organischen Lösungsmitteln wie zum Beispiel Benzol, Toluol oder Acetonitril, unter Schutzgasatmosphäre bei Reaktionstemperaturen zwischen 10°C und der Siedetemperatur des eingesetzten Lösungsmittels zu Verbindungen der allgemeinen Formel VII 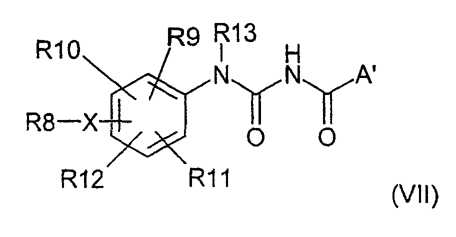 in denen X, R8, R9, R10, R11, R12, R13 und A' die oben beschrieben Bedeutung haben,
umgesetzt,
die Verbindungen der allgemeinen Formel VII können, falls R1 in Verbindungen der allgemeinen Formel I kein Wasserstoffatom darstellt, durch Umsetzung mit Verbindungen der allgemeinen Formel VIII

R14-LG (VIII)

in denen LG die oben beschriebene Bedeutung hat und
- R14: H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COO-(PG-1), (C₁-C₆)-Alkylen-COO-(C₁-C₆)-alkyl
wobei PG-1 die oben beschriebene Bedeutung hat,
unter Verwendung einer Base, wie zum Beispiel 1,8-Diazabicyclo[5.4.0]undec-7-en, in organischen Lösungsmitteln, wie zum Beispiel Dichlormethan oder Acetonitril, zu Verbindungen der allgemeinen Formel IX 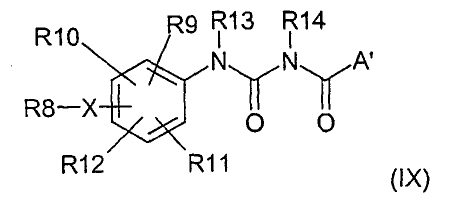 in denen X, R8, R9, R10, R11, R12, R13, R14 und A' die oben beschrieben Bedeutung haben,
alkyliert werden,
und nach literaturbekannter Abspaltung aller eventuell vorhandenen Schutzgruppen in den Resten R8, R9, R10, R11, R12, R13, R14, A' und B' erhält man Verbindungen der allgemeinen Formel I. Die Überführung der Verbindungen der allgemeinen Formel I in deren Salze erfolgt durch Zugabe eines Äquivalentes der entsprechenden Säure oder Base in einem organischen Lösungsmittel wie zum Beispiel Acetonitril oder Dioxan oder in Wasser und durch anschließende Entfernung des Lösungsmittels.

Eine weitere Möglichkeit Verbindungen der allgemeinen Formel I, in denen R2 ein Wasserstoffatom darstellt, herzustellen ist im folgenden Schema dargestellt: 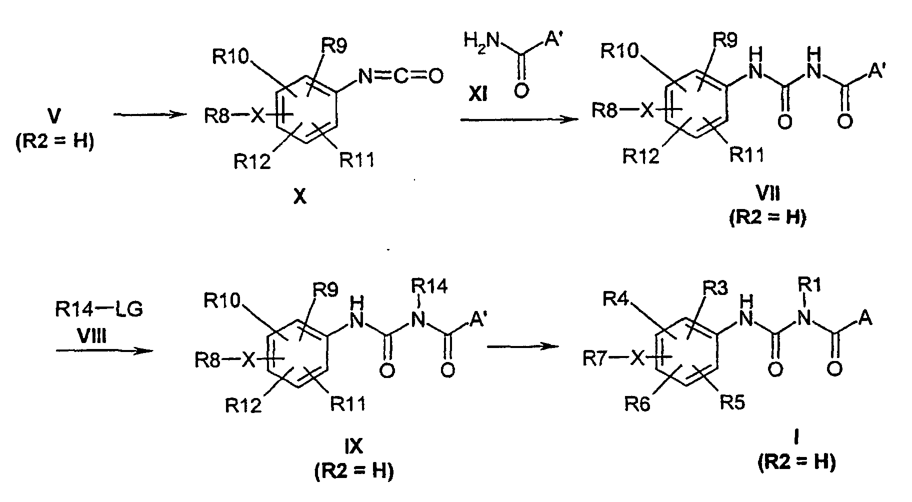 dabei werden Verbindungen der allgemeinen Formel V, in denen R2 ein Wasserstoffatom darstellt, 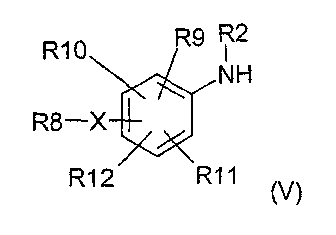 und X, R8, R9, R19, R11 und R12 die oben beschriebene Bedeutung haben, in Isocyanate der allgemeinen Formel X 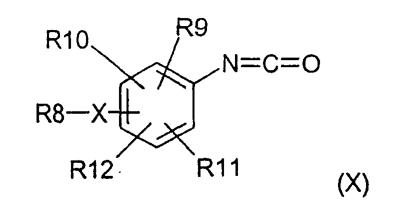 nach bekannten Methoden, wie zum Beispiel der Umsetzung mit Oxalylchlorid in organischen Lösungsmitteln, wie zum Beispiel 1,2-Dichlorethan oder Dichlormethan, bei Reaktionstemperaturen zwischen Raumtemperatur und dem Siedepunkt des Lösungsmittels umgewandelt,
die Isocyanate der allgemeinen Formel X bringt man mit Amiden der allgemeinen Formel XI 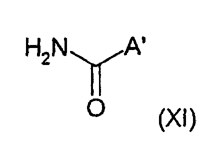 in denen A' die oben beschriebene Bedeutung hat,
zur Reaktion und erhält Verbindungen der allgemeinen Formel VII in denen R2 ein Wasserstoffatom darstellt, 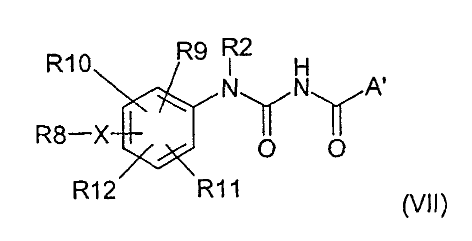 und X, R8, R9, R10, R11 und R12 die oben beschriebene Bedeutung haben, Verbindungen der allgemeinen Formel VII können, wenn R1 kein Wasserstoffatom darstellt, wie bereits oben beschrieben durch Alkylierung mit Verbindungen der allgemeinen Formel VIII zu Verbindungen der allgemeinen Formel IX, und, falls notwendig, durch anschließende Abspaltung der Schutzgruppen in Verbindungen der allgemeinen Formel I überführt werden. Die Überführung der Verbindungen der allgemeinen Formel I in deren Salze erfolgt durch Zugabe eines Äquivalentes der entsprechenden Säure oder Base in einem organischen Lösungsmittel wie zum Beispiel Acetonitril oder Dioxan oder in Wasser und durch anschließende Entfernung des Lösungsmittels.

Die nachfolgend aufgeführten Beispiele dienen zur Erläuterung der Erfindung, ohne diese jedoch einzuschränken. Die gemessenen Fest-, bzw. Zersetungspunkte (Fp.) wurden nicht korrigiert und sind generell von der Aufheizgeschwindigkeit abhängig.

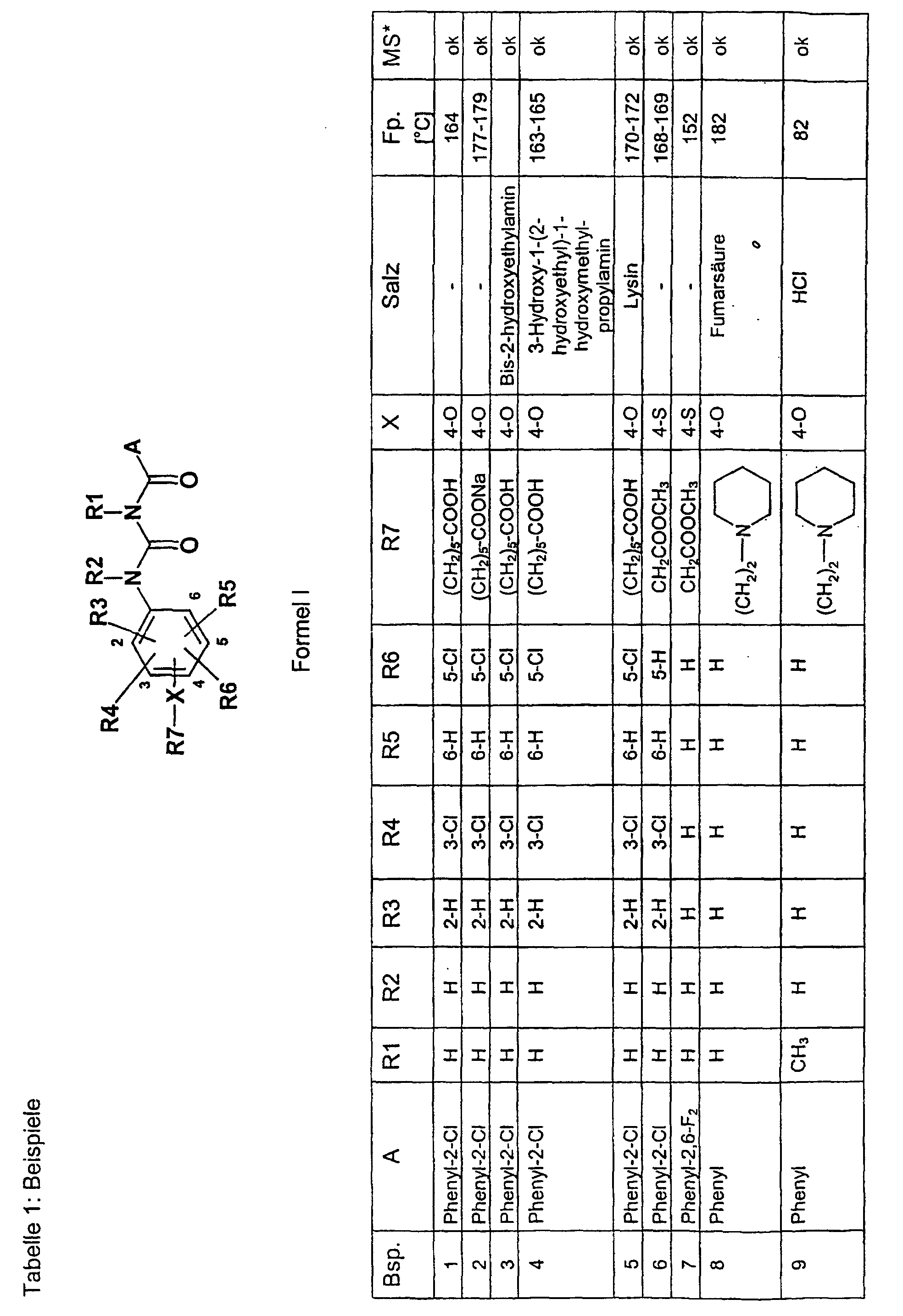

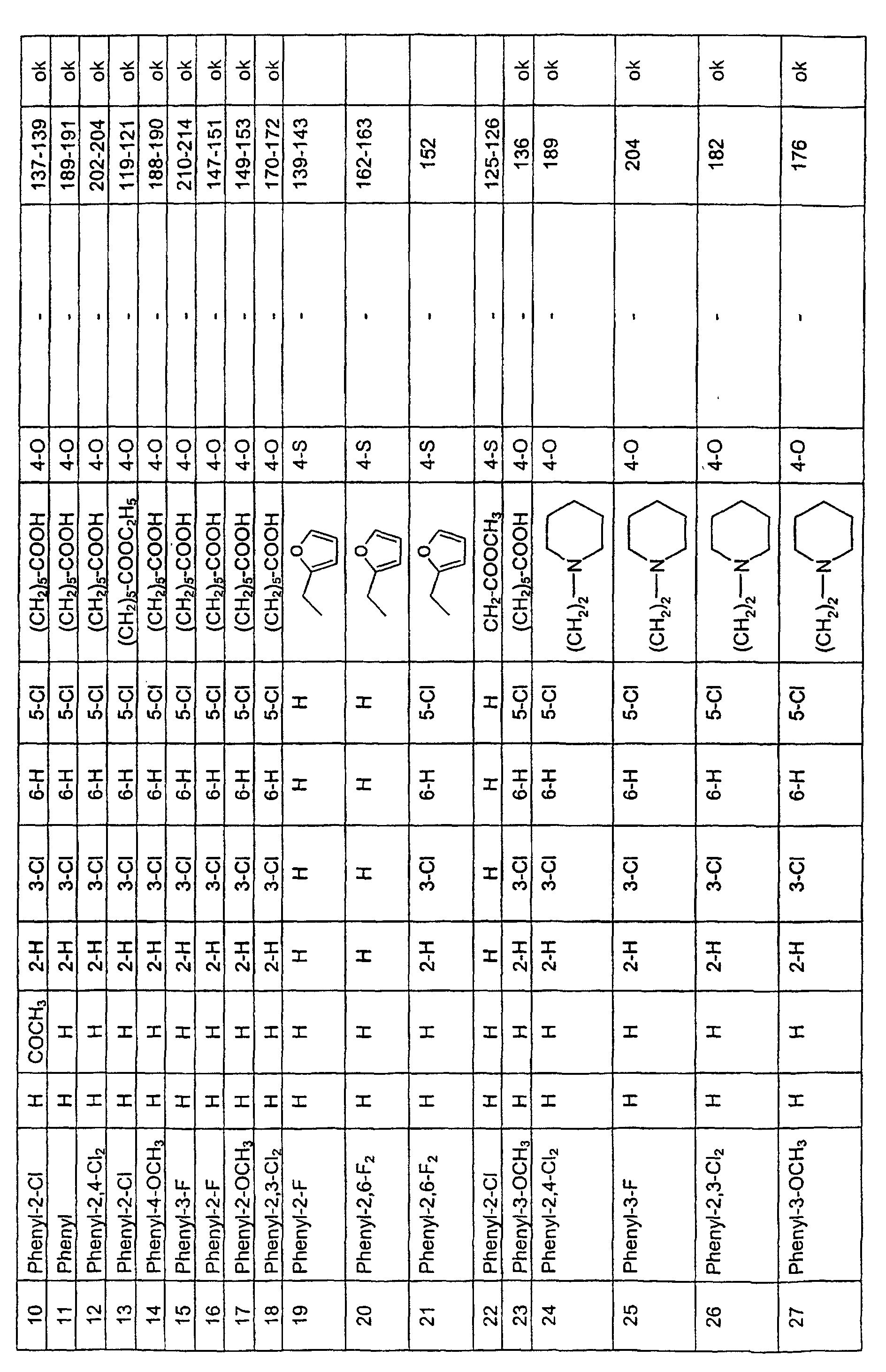

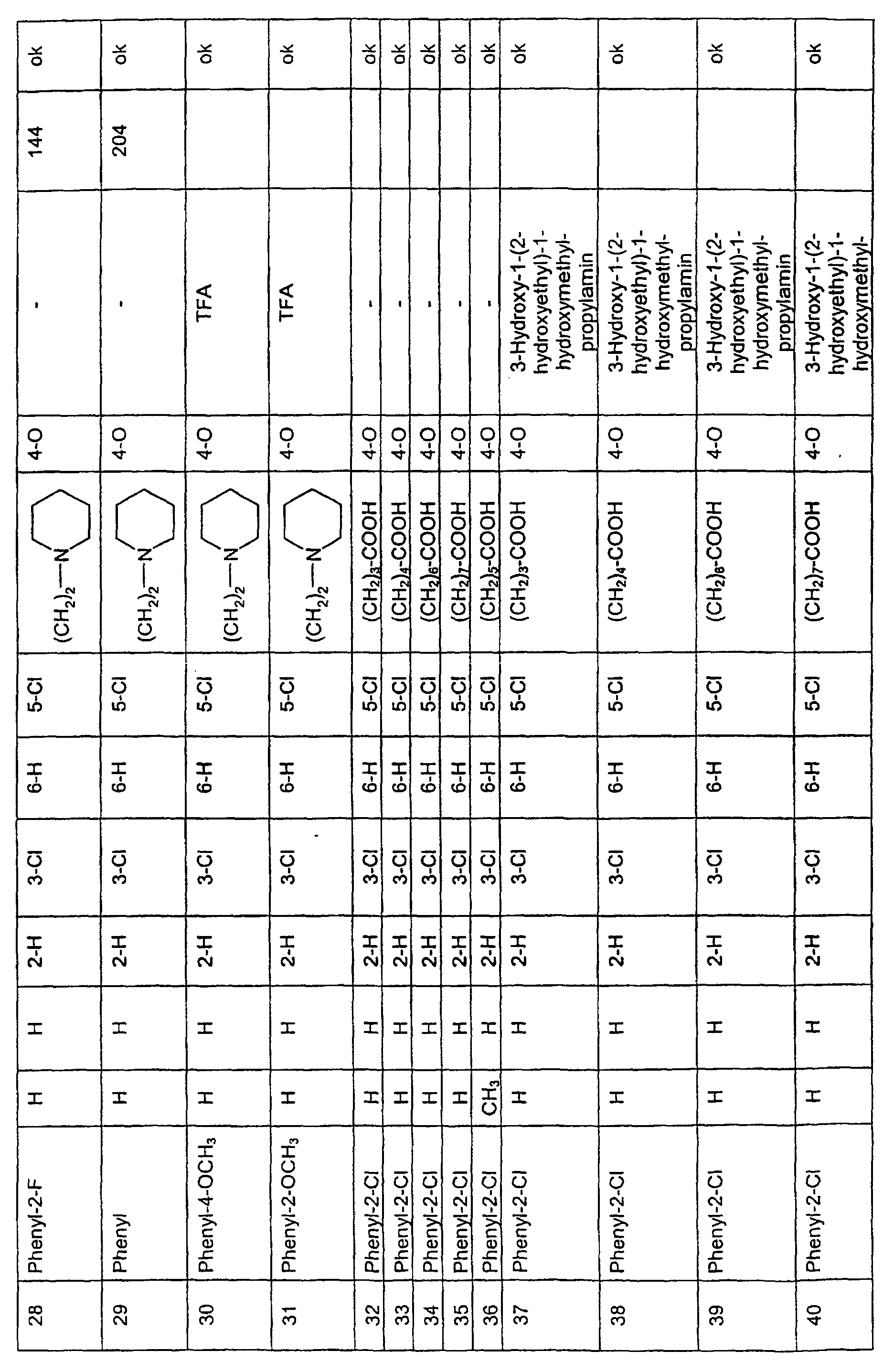

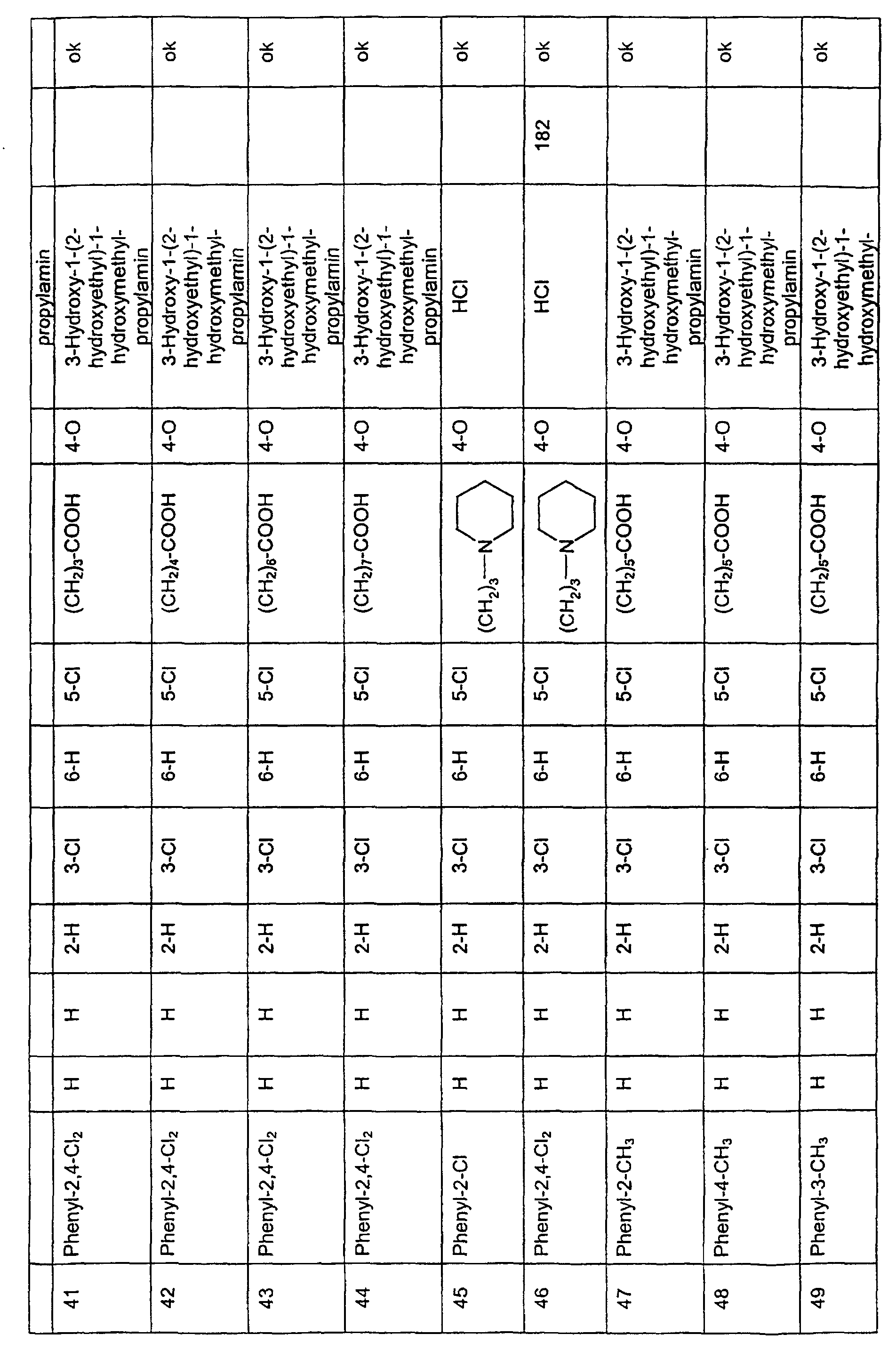

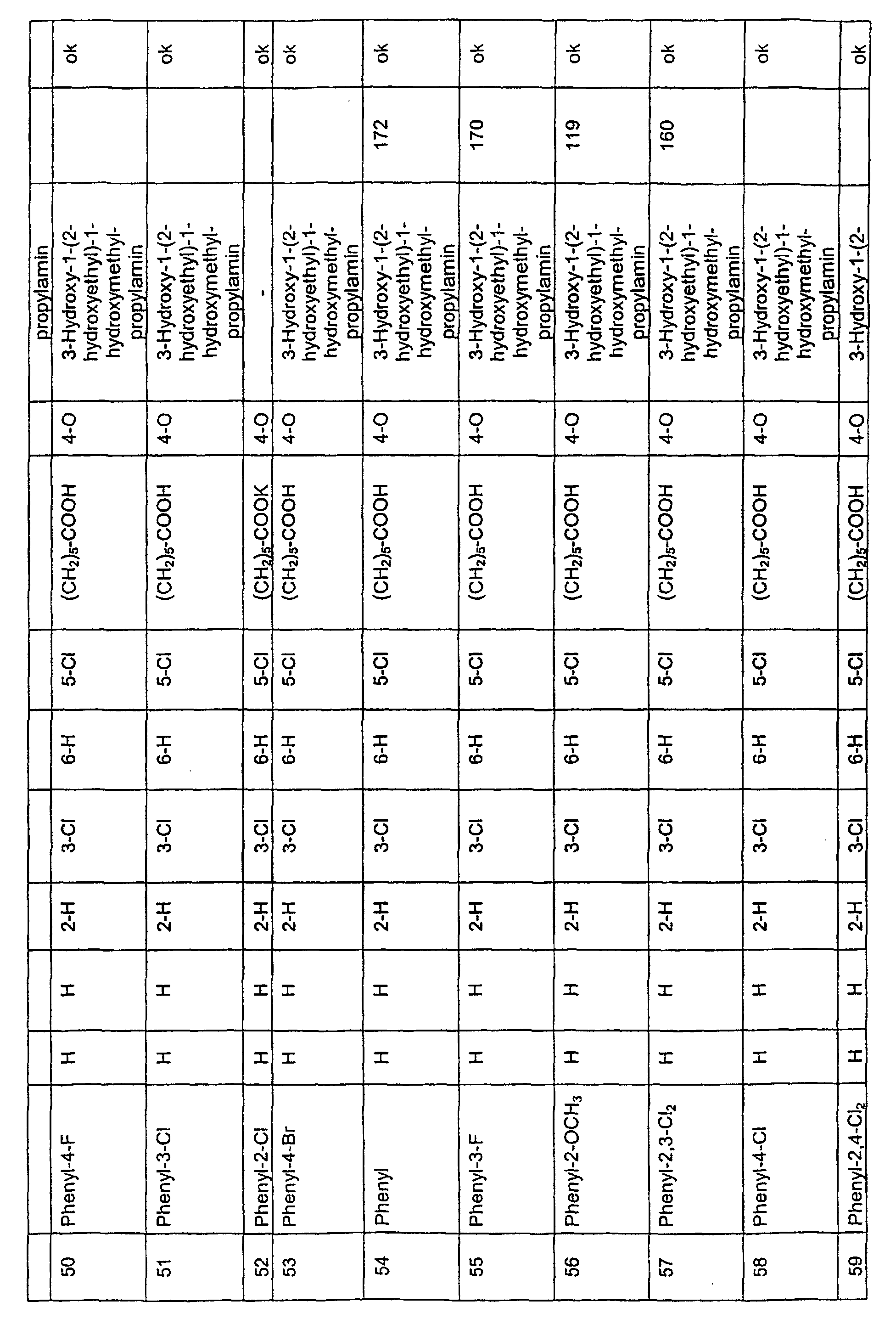

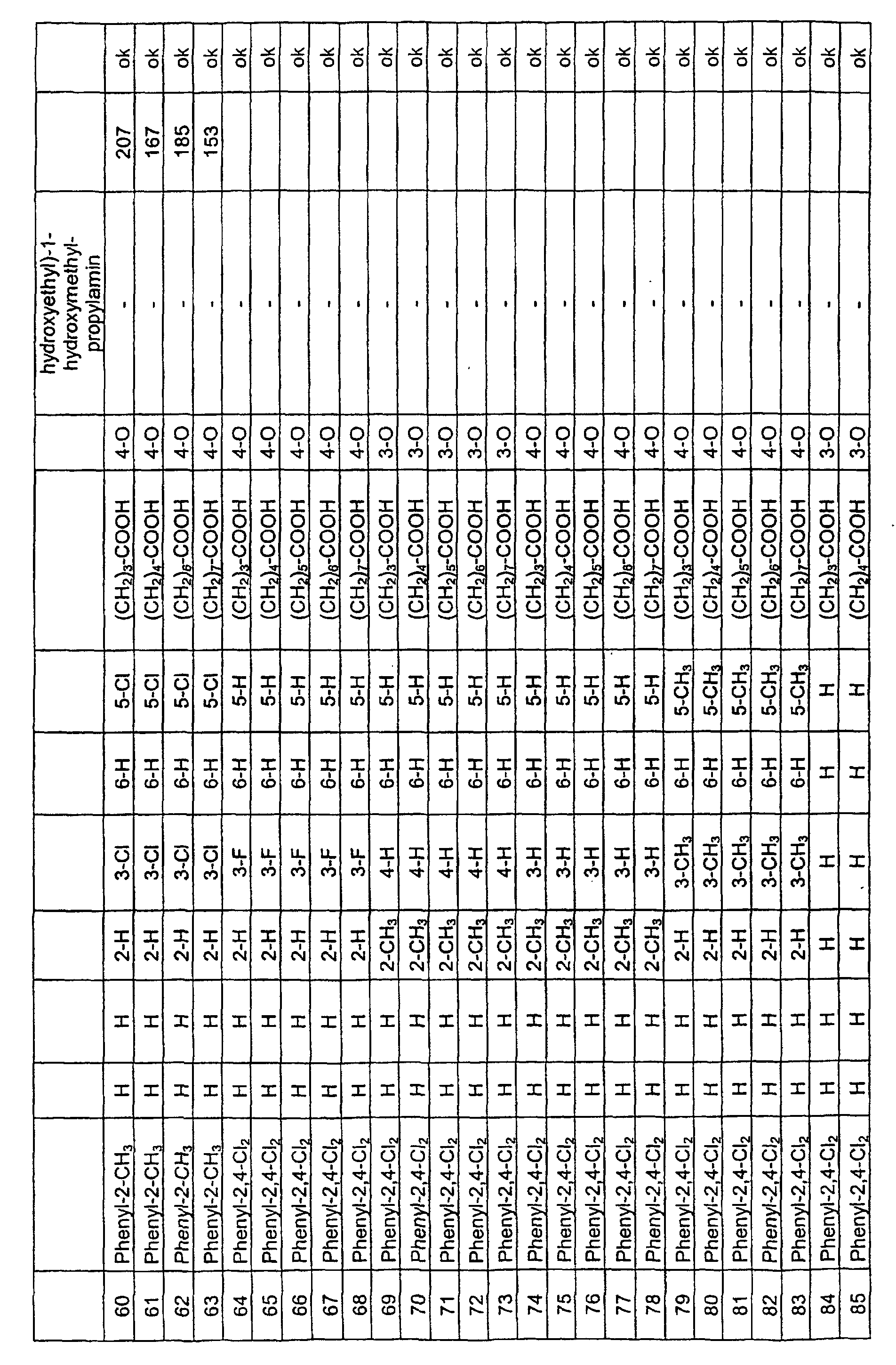

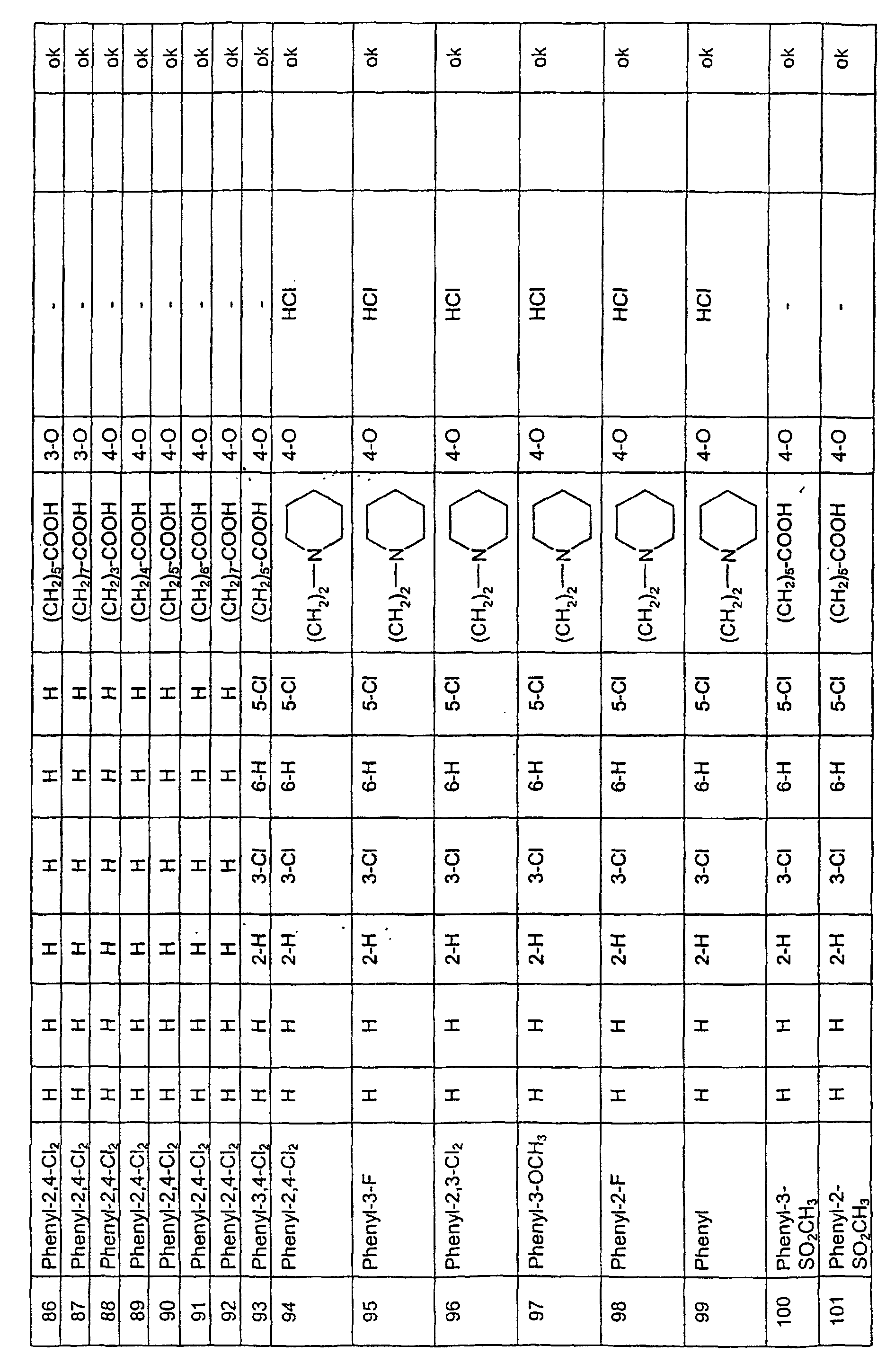

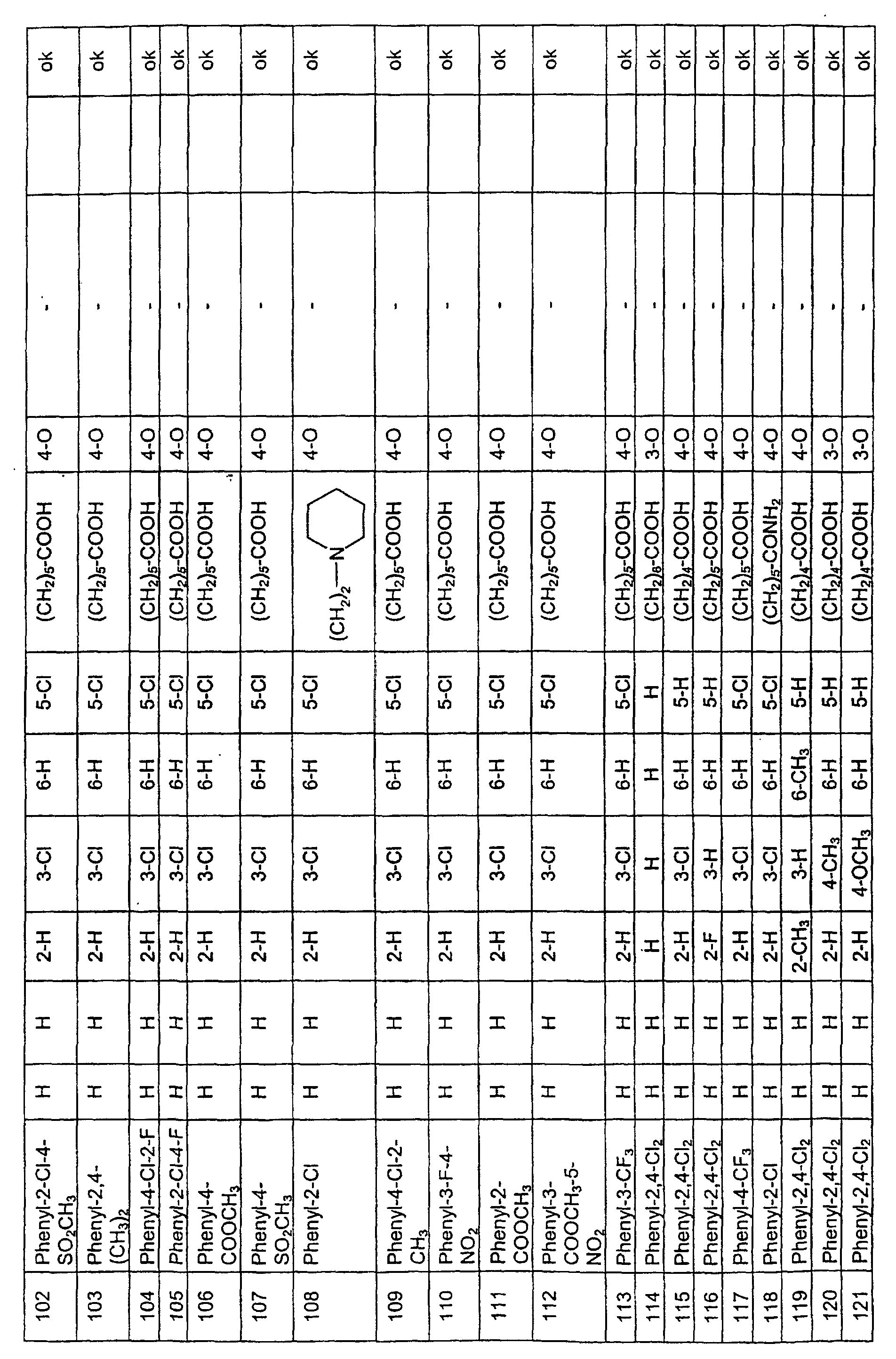

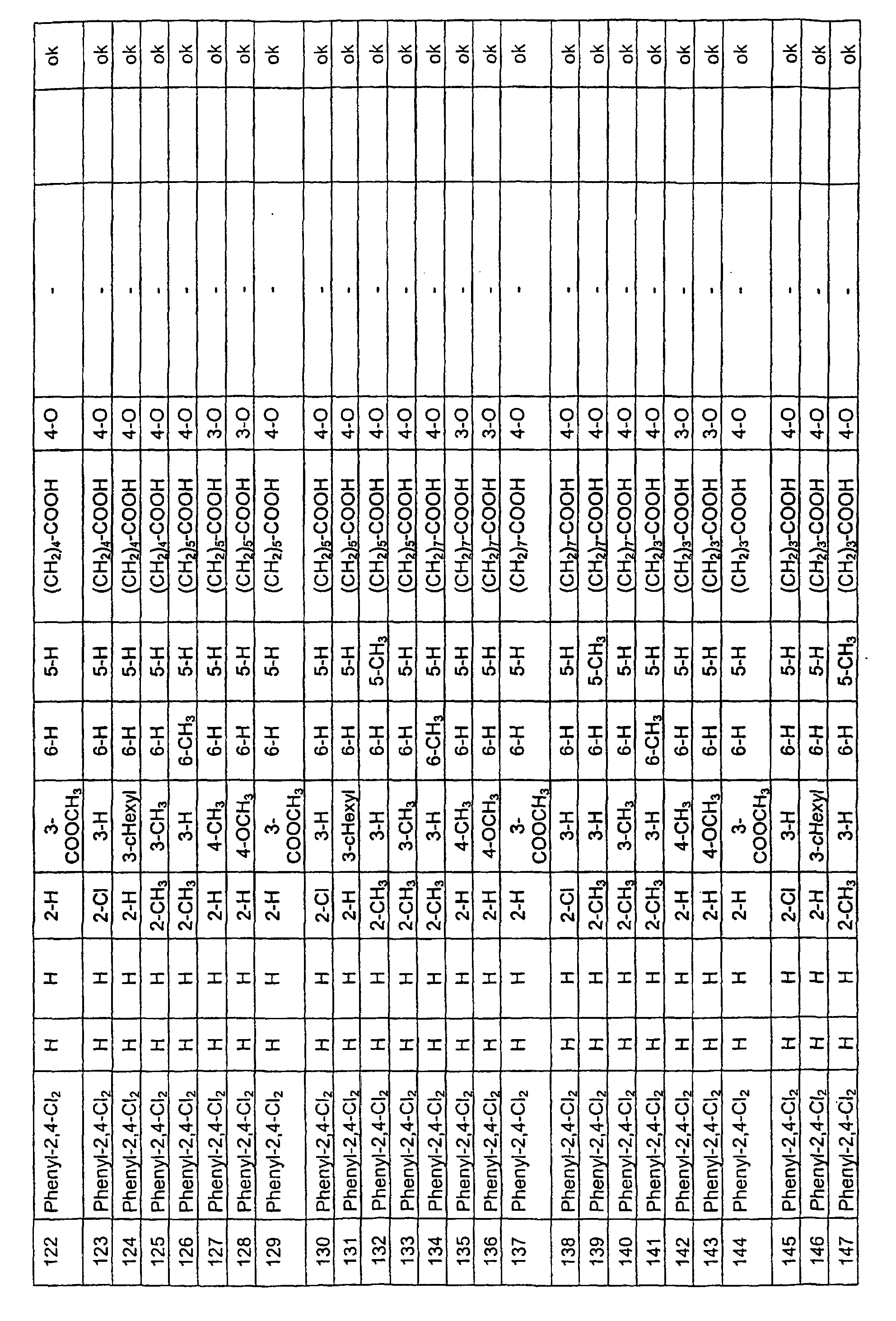

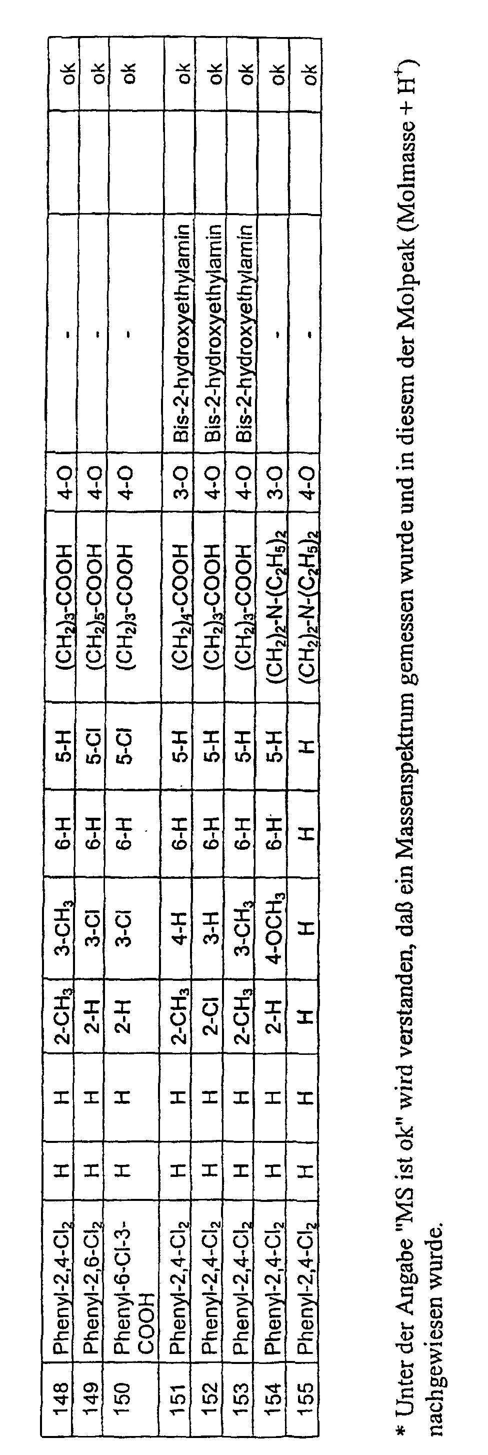

Die Verbindungen der Formel I zeichnen sich durch günstige Wirkungen auf den Zuckerstoffwechsel aus, sie senken insbesondere den Blutzuckerspiegel und sind zur Behandlung von Typ II Diabetes geeignet. Die Verbindungen können allein oder in Kombination mit weiteren Blutzucker senkenden Wirkstoffen eingesetzt werden. Solche weiteren Blutzucker senkenden Wirkstoffe sind zum Beispiel Sulfonylharnstoffe (wie zum Beispiel Glimepirid, Glibenclamid), Glitazone (wie zum Beispiel Troglitazon, Rosiglitazon), alpha-Glucosidase-Hemmer (wie zum Beispiel Acarbose, Miglitol) oder Insuline.

Die Wirksamkeit der Verbindungen wurde wie folgt getestet:

### Glykogenphophorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde. Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCI, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (al (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.
Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2:**

| Biologische Aktivität | |
|---|---|
| Bsp. | % Hemmung bei 10 µM |
| 1 | 87 |
| 2 | 73 |
| 3 | 75 |
| 4 | 79 |
| 5 | 77 |
| 12 | 92 |
| 20 | 35 |
| 29 | 78 |
| 30 | 76 |
| 31 | 86 |
| 41 | 50 |
| 44 | 11 |
| 46 | 36 |
| 47 | 46 |
| 49 | 13 |
| 51 | 36 |
| 53 | 22 |
| 60 | 36 |
| 70 | 86 |
| 75 | 41 |
| 80 | 50 |
| 84 | 44 |
| 89 | 90 |
| 90 | 34 |
| 100 | 78 |
| 101 | 93 |
| 102 | 14 |
| 106 | 35 |
| 111 | 88 |
| 112 | 100 |
| 116 | 100 |
| 117 | 99 |
| 118 | 70 |
| 119 | 97 |
| 120 | 40 |
| 122 | 12 |
| 128 | 95 |
| 147 | 88 |
| 149 | 76 |

Aus der Tabelle ist abzulesen, daß die Verbindungen der Formel I die Aktivität der Glykogenphosphorylase a hemmen und dadurch zur Senkung des Blutzuckerspiegels gut geeignet sind.

Nachfolgend wird die Herstellung einiger Beispiele detailliert beschrieben, die übrigen Verbindungen der Formel I wurden analog erhalten:
Experimenteller Teil:

### Beispiel 1:

### 6-{2,6-Dichloro-4-[(2-chloro-benzoyl)-aminocarbamoyl]-phenoxy}-hexansäure

### a) 6-(4-Acetylamino-2,6-dichloro-phenoxy)-hexansäureethylester

Zu einer Lösung von 15,0 g (68,1 mmol) N-(3,5-Dichloro-4-hydroxy-phenyl)-acetamid in 300 ml Aceton gibt man 13,3 ml (74,9 mmol) 6-Brom-hexansäureethylester und 52,1 g (160 mmol) Cäsiumkarbonat. Die Suspension wird 8 Stunden unter Rückfluß gekocht. Anschließend gibt man 600 ml Wasser hinzu, extrahiert zwei mal mit je 400 ml Dichlormethan und mit 400 ml MTB-Ether. Die vereinigten organische Phasen werden mit Wasser gewaschen und am Rotationsverdampfer eingeengt. Das Produkt wird ohne Reinigung im nächsten Schritt eingesetzt. Rohausbeute: 30 g

### b) 6-(4-Acetylamino-2,6-dichloro-phenoxy)-hexansäure

30 g Rohmaterial aus Stufe a) werden mit 800 ml 1 m Kaliumhydroxid-Lösung versetzt und 3 Tage bei Raumtemperatur gerührt. Anschließend werden 600 ml Wasser zugegeben und mit ca 80 ml Eisessig auf pH 5,5 gebracht. Das ausgefallene Produkt wird abgesaugt und zwei mal mit je 40 ml Wasser nachgewaschen. Der Niederschlag wird im Hochvakuum getrocknet und ergibt 14,6 g der gewünschten Verbindung.

### c) 6-(4-Amino-2,6-dichloro-phenoxy)-hexansäure

7,5 g (22,4 mmol) 6-(4-Acetylamino-2,6-dichloro-phenoxy)-hexansäure in 140 ml 1 m Kaliumhydroxid-Lösung in Methanol-Wasser (3:1) über Nacht unter Rückfluß gekocht. Das Methanol wird am Rotationsverdampfer entfernt, der Rückstand wird mit ca 30 ml Wasser verdünnt und mit Eisessig auf pH 5 angesäuert. Das Gemisch wird 30 Minuten im Eisbad gerührt und anschließend abgesaugt. Das Rohprodukt wird säulenchromatographisch unter Verwendung von n-Heptan /Essigsäureethylester = 1/1 getrennt und ergibt 4,3 g (14,7 mmol, 66%) des gewünschten Produkts.

### d) 6-{2,6-Dichloro-4-[(2-chloro-benzoyl)-aminocarbamoyl]-phenoxy}-hexansäure

Zu einer Suspension von 10,0 g (34,2 mmol) 6-(4-Amino-2,6-dichloro-phenoxy)-hexansäure werden in 700 ml trockenem Acetonitril unter Schutzgasatmosphäre bei Raumtemperatur eine Lösung von 7,5 g (41,1 mmol) 2-Chlorbenzoylisocyanat in 300 ml Acetonitril zugegeben. Es wird 2 Stunden unter Rückfluß gekocht und auf Raumtemperatur abgekühlt. Der entstandenen Niederschlag wird abgesaugt und mit 50 ml Acetonitril nachgewaschen. Der Rückstand wird mit 100 ml Methanol verrührt, abgesaugt, mit wenig Methanol nachgewaschen und bei 40°C unter Vakuum über Nacht getrocknet. Man erhält 13,7 g (28,9 mmol, 85 %) des gewünschten Produktes. Schmelzpunkt: 171-173°C

## Patentansprüche

1. Verbindungen der Formel I, 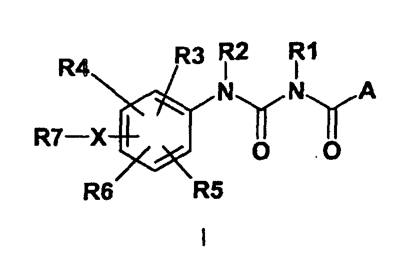 worin bedeuten
A Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO(C₁-C₇)-alkyl, CONH₂, CONH(C₁-C₆)-Alkyl, CON[(C₁-C₆)-Alkyl]₂, CONH(C₃-C₆)-Cycloalkyl, (C₀-C₆)-Alkylen-NH₂, (C₀-C₆)-Alkylen-NH(C₂-C₆)-alkyl, (C₀-C₆)-Alkylen-N[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
R1, R2 unabhängig voneinander H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COOH, (C₁-C₆)-Alkylen-COO-(C₁-C₆)-alkyl;
R3, R4, R5, R6 unabhängig voneinander H, F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, COOH, COO(C₁-C₆)-Alkyl, CONH₂, CONH(C₁-C₆)-Alkyl, CON[(C₁-C₆)-Alkyl]₂, CONH(C₃-C₇)-Cycloalkyl, NH₂, NH(C₁-C₆)-Alkyl, N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
X O, S;
R7 (C₁-C₁₀)-Alkylen-COOH, (C₆-C₁₀)-Alkylen-COO-(C₁-C₆)-Alkyl, (C₁-C₁₀)-Alkylen-CONH₂, (C₁-C₁₀)-Alkylen-CONH-(C₁-C₆)-alkyl, (C₁-C₁₀)-Alkylen-CON-[(C₁-C₆)-alkyl]₂, (C₁-C₁₀)-Alkylen-NH₂, (C₁-C₁₀)-Alkylen-NH(C₁-C₆)-Alkyl, (C₁-C₁₀)-Alkylen-N[(C₁-C₆)-Alkyl]₂, (C₁-C₁₀)-Alkylen-B;
B (C₃-C₇)-Cycloalkyl, Pyrrolyl, Imidazolyl, Thiazolyl, Azetidinyl, Thienyl, Piperidinyl, Pyrrolidinyl, Morpholinyl, Pyridyl-methyl oder Furyl, worin Cycloalkyl, Pyrrolyl, Imidazolyl, Thiazolyl, Azetidinyl, Thienyl, Piperidinyl, Pyrrolidinyl, Morpholinyl, Pyridyl oder Furyl jeweils bis zu zweimal mit Cl, F, CN, CF₃, OCF₃, COOH, COO-(C₁-C₆)-Alkyl, CONH₂, CONH-(C₁-C₆)-Alkyl, CON-[(C₁-C₆)-Alkyl]₂, (C₁-C₆)-Alkyl, OH, O-(C₁-C₆)-Alkyl substituiert sein können;
sowie deren physiologisch verträgliche Salze,
wobei die Verbindung der Formel 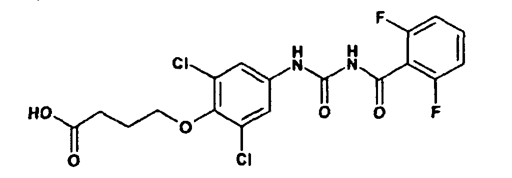 sowie Verbindungen der Formel I, worin die Reste gleichzeitig bedeuten
A Phenyl;
X O;
R1 H;
R7 -(C₁-C₄)-Alkyl-B;
B (C₃-C₇)-Cycloalkyl, Heteroaryl;
ausgenommen sind.

2. Verbindungen der Formel I, gemäß Anspruch 1, **dadurch gekennzeichnet, daß** darin bedeuten
A Phenyl, wobei der Phenylrest bis zu zweifach substituiert sein kann mit F, Cl, Br, O-(C₁-C₆)-Alkyl;
R1, R2 unabhängig voneinander H, (C₁-C₆)-Alkyl, CO-(C₁-C₆)-Alkyl;
R3, R4, R5, R6 unabhängig voneinander H, Cl, F, (C₁-C₆)-Alkyl, O-(C₁-C₆)-Alkyl, -COO-(C₁-C₆)-Alkyl;
X O;
R7 (C₁-C₁₀)-Alkylen-COOH, (C₆-C₁₀)-Alkylen-COO-(C₁-C₆)-Alkyl, (C₁-C₁₀)-Alkylen-CONH₂;
sowie deren physiologisch verträgliche Salze,
wobei die Verbindung der Formel 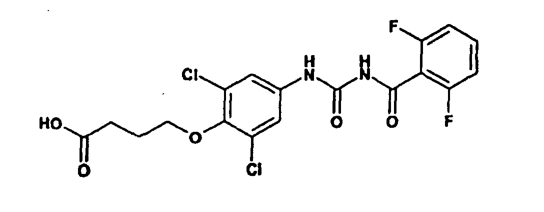 ausgenommen ist.

3. Arzneimittel enthaltend eine oder mehrere der Verbindungen gemäß Anspruch 1 oder 2.

4. Arzneimittel enthaltend eine oder mehrere der Verbindungen gemäß Anspruch 1 oder 2 und ein oder mehrere Blutzucker senkende Wirkstoffe.

5. Verwendung der Verbindungen gemäß Anspruch 1 oder 2 zur Herstellung eines Medikamentes.

6. Verwendung der Verbindungen gemäß Anspruch 1 oder 2 zur Herstellung eines Medikamentes zur Blutzuckersenkung.

7. Verwendung der Verbindungen gemäß Anspruch 1 oder 2 zur Herstellung eines Medikamentes zur Behandlung des Typ II Diabetes.

8. Verwendung der Verbindungen gemäß Anspruch 1 oder 2 in Kombination mit mindestens einem weiteren Blutzucker senkenden Wirkstoff zur Herstellung eines Medikamentes.

9. Verfahren zur Herstellung eines Arzneimittels enthaltend eine oder mehrere der Verbindungen gemäß Anspruch 1 oder 2, **dadurch gekennzeichnet, daß** der Wirkstoff mit einem pharmazeutisch geeigneten Träger vermischt wird und diese Mischung in eine für die Verabreichung geeignete Form gebracht wird.

10. Verwendung der Verbindung der Formel I 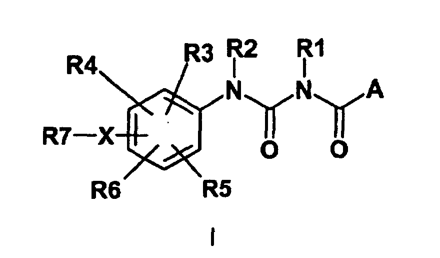 worin bedeuten
A Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO(C₁-C₆)-alkyl, CONH₂, CONH(C₁-C₆)-Alkyl, CON[(C₁-C₆)-Alkyl]₂, CONH(C₃-C₇)-Cycloalkyl, (C₀-C₆)-Alkylen-NH₂, (C₀-C₆)-Alkylen-NH(C₁-C₆)-Alkyl, (C₀-C₆)-Alkylen-N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
R1, R2 unabhängig voneinander H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COOH, (C₁-C₆)-Alkylen-COO-(C₁-C₆)-alkyl;
R3, R4, R5, R6 unabhängig voneinander H, F, Cl, Br, OH, CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-NH₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, COOH, COO(C₁-C₆)-Alkyl, CONH₂, CONH(C₁-C₆)-Alkyl, CON[(C₁-C₆)-Alkyl]₂, CONH(C₃-C₇)-Cycloalkyl, NH₂, NH(C₁-C₆)-Alkyl, N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO(C₁-C₆)-Alkyl oder CONH₂ substituiert sein kann;
X O, S;
R7 (C₁-C₁₀)-Alkylen-COOH, (C₁-C₁₀)-Alkylen-COO-(C₁-C₆)-alkyl, (C₁-C₁₀)-Alkylen-CONH₂, (C₁-C₁₀)-Alkylen-CONH-(C₁-C₆)-alkyl, (C₁-C₁₀)-Alkylen-CON-[(C₁-C₆)-alkyl]₂, (C₁-C₁₀)-Alkylen-NH₂, (C₁-C₁₀)-Alkylen-NH(C₁-C₆)-alkyl, (C₁-C₁₀)-Alkylen-N[(C₁-C₆)-alkyl]₂, (C₁-C₁₀)-Alkylen-B;
B (C₃-C₇)-Cycloalkyl, Phenyl, Pyrrolyl, Imidazolyl, Thiazolyl, Azetidinyl, Thienyl, Piperidinyl, Pyrrolidinyl, Morpholinyl, Pyridyl oder Furyl, worin Cycloalkyl, Phenyl, Pyrrolyl, Imidazolyl, Thiazolyl, Azetidinyl, Thienyl, Piperidinyl, Pyrrolidinyl, Morpholinyl, Pyridyl oder Furyl jeweils bis zu zweimal mit Cl, F, CN, CF₃, OCF₃, COOH, COO-(C₁-C₆)-Alkyl, CONH₂, CONH-(C₁-C₆)-Alkyl, CON-[(C₁-C₆)-Alkyl]₂ (C₁-C₆)-Alkyl, OH, O-(C₁-C₆)-Alkyl substituiert sein können;
sowie deren physiologisch verträgliche Salze zur Herstellung eines Medikamentes zur Senkung des Blutzuckerspiegels.

11. Verfahren zur Herstellung der Verbindungen gemäß Anspruch 1 oder 2, **dadurch gekennzeichnet, daß** man nach folgendem Formelschema 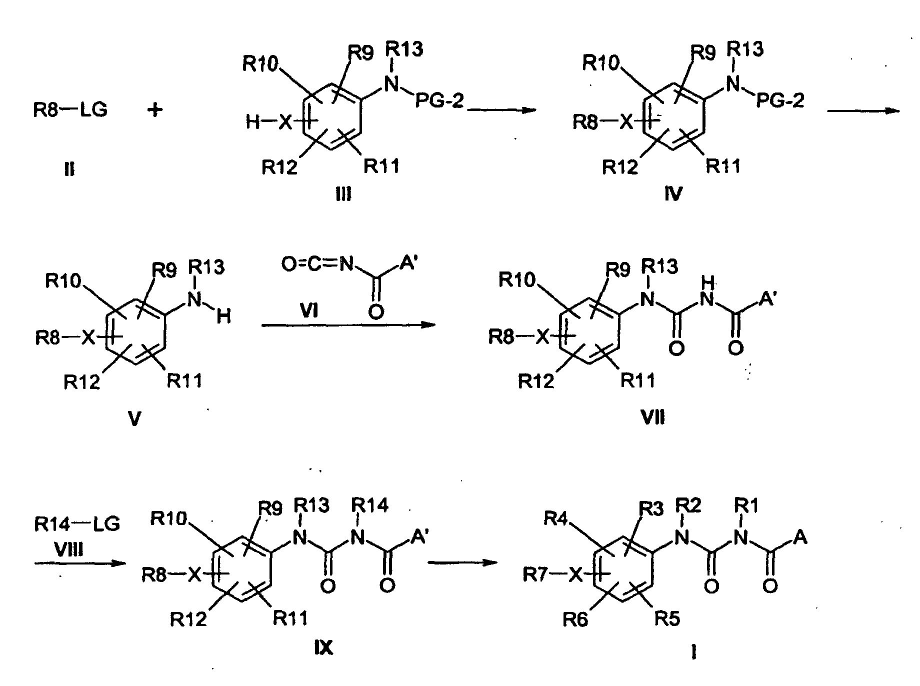 die Verbindung der allgemeinen Formel 11,
R8-LG (II)
worin bedeuten
R8 (C₁-C₁₀)-Alkylen-COO-(PG-1), (C₆-C₁₀)-Alkylen-COO-(C₁-C₆)-alkyl, (C₁-C₁₀)-Alkylen-CON-(PG-2)₂, (C₁-C₁₀)-Alkylen-CONH-(C₁-C₆)-alkyl, (C₁-C₁₀)-Alkylen-CON-[(C₁-C₆)-alkyl]₂, (C₁-C₁₀)-Alkylen-N-(PG-2)₂, (C₁-C₁₀)-Alkylen-NH(C₁-C₆)-alkyl, (C₁-C₁₀)-Alkylen-N[(C₁-C₆)-alkyl]₂, (C₁-C₁₀)-Alkylen-B'
PG-1 eine allgemein bekannte Schutzgruppe für Ester, wie zum Beispiel (C₁-C₆)-Alkyl, Benzyl oder p-Methoxybenzyl;
PG-2 eine allgemein bekannte Schutzgruppe für Aminogruppen, wie zum Beispiel (C₁-C₆)-Alkylcarbonyl, (C₁-C₆)-Alkyloxycarbonyl oder (C₆-C₁₂)-Aryl-(C₁-C₄)-alkyloxycarbonyl, die entweder beide Wasserstoffe oder nur ein Wasserstoffatom der Aminogruppe ersetzt;
B' (C₃-C₇)-Cycloalkyl, Pyrrolyl, Imidazolyl, Thiazolyl, Azetidinyl, Thienyl, Piperidinyl, Pyrrolidinyl, Morpholinyl, Pyridyl-methyl und Furyl, worin Cycloalkyl, Pyrrolyl, Imidazolyl, Thiazolyl, Azetidinyl, Thienyl, Piperidinyl, Pyrrolidinyl, Morpholinyl, Pyridyl und Furyl jeweils bis zu zweimal mit Cl, F, CN, CF₃, OCF₃, COO-(PG-1), COO-(C₁-C₆)-Alkyl, CON-(PG-2)₂, CONH-(C₁-C₆)-Alkyl, CON-[(C₁-C₆)-Alkyl]₂, (C₁-C₆)-Alkyl, O-(PG-3), O-(C₁-C₆)-Alkyl substituiert sein können;
PG-3 eine allgemein bekannte Schutzgruppe für Alkohole, wie zum Beispiel Benzyl, Allyl, Tetrahydropyranyl oder Tetrahydrofuranyl;
LG eine allgemein bekannte Austrittsgruppe, wie zum Beispiel Halogen, Arylsulfonyloxy oder Alkylsulfonyloxy;
mit Anilinen der allgemeinen Formel III 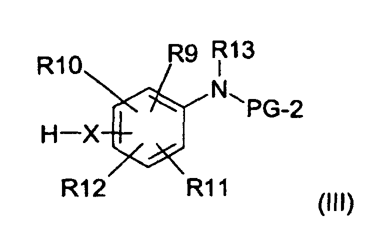 worin X und PG-2 die oben beschriebene Bedeutung haben und worin bedeuten
R9, R10, R11, R12 unabhängig voneinander H, F, Cl, Br, O-(PG-3), CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-N-(PG-2)₂, (C₁-C₆)-Alkyl, (C₁-C₆)-< Alkenyl, (C₁-C₆)-Alkinyl, (C₃-C₇)-Cycloalkyl, (C₃-C₇)-Cycloalkyl-(C₁-C₄)-alkylen, COO-(PG-1), COO(C₁-C₆)-Alkyl, CON-(PG-2)₂, CONH(C₁-C₆)-Alkyl, CON[(C₁-C₆)-Alkyl]₂, CONH(C₃-C₇)-Cycloalkyl, N-(PG-2)₂, NH(C₁-C₆)-Alkyl, N[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, O-(PG-3), (C₁-C₆)-Alkyl, O-(C₁-C₆)-Alkyl, CF₃, OCF₃, COO-(PG-1), COO(C₁-C₆)-Alkyl oder CON-(PG-2)₂ substituiert sein kann;
R13 H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COO-(PG-1), (C₁-C₆)-Alkylen-COO-(C₁-C₆)-alkyl darstellt;
worin PG-1, PG-2 und PG-3 die oben beschriebene Bedeutung haben;
unter Verwendung einer Base, wie zum Beispiel Kalium- oder Cäsiumkarbonat, in einem organische Lösungsmittel, wie zum Beispiel Aceton oder Dimethylformamid,
zu Verbindungen der allgemeinen Formel IV 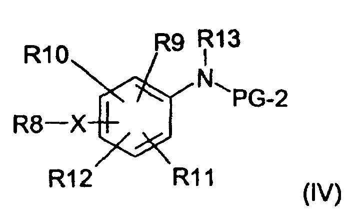 in denen X, R8, R9, R10, R11, R12, R13 und PG-2 die oben beschrieben Bedeutung haben,
alkyliert
und diese anschließend durch selektive Abspaltung der Schutzgruppe PG-2 zu Verbindungen der allgemeinen Formel V 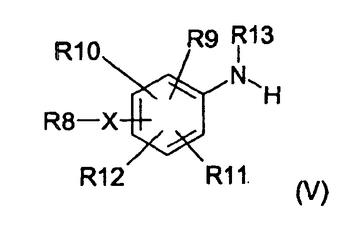 worin X, R8, R9, R10, R11, R12, und R13 die oben aufgeführten Bedeutungen haben;
umgesetzt und diese dann mit Isocyanaten der allgemeinen Formel VI 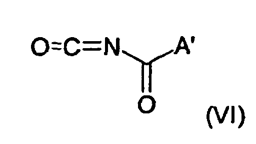 worin bedeuten
A' Phenyl, Naphthyl, wobei der Phenyl- oder Naphthylrest bis zu dreifach substituiert sein kann mit F, CI, Br, O-(PG-3), CF₃, NO₂, CN, OCF₃, O-(C₁-C₆)-Alkyl, O-(C₁-C₆)-Alkenyl, O-(C₁-C₆)-Alkinyl, S-(C₁-C₆)-Alkyl, S-(C₁-C₆)-Alkenyl, S-(C₁-C₆)-Alkinyl, SO-(C₁-C₆)-Alkyl, SO₂-(C₁-C₆)-Alkyl, SO₂-N-(PG-2)₂, (C₁-C₆)-Alkyl, (C₁-C₆)-Alkenyl, (C₁-C₆)-Alkinyl, (C₃-C₇)-Cycloalkyl, (C₃-C₇)-Cycloalkyl-(C₁-C₄)-alkylen, (C₀-C₆)-Alkylen-COO-(PG-1), (C₀-C₆)-Alkylen-COO(C₁-C₆)-alkyl, CON-(PG-2)₂, CONH(C₁-C₆)-Alkyl, CON[(C₁-C₆)-Alkyl]₂, CONH(C₃-C₇)-Cycloalkyl, (C₀-C₆)-Alkylen-N-(PG-2)₂, (C₀-C₆)-Alkylen-NH(C₁-C₆)-alkyl, (C₀-C₆)-Alkylen-N[(C₁-C₆)-alkyl]₂, NH-CO-(C₁-C₆)-Alkyl, NH-CO-Phenyl, NH-SO₂-Phenyl, wobei der Phenylring bis zu zweifach mit F, CI, CN, O-(PG-3), (C₁-C₆)-Alkyl, O-(C₁-C₆)-Alkyl, CF₃, OCF₃, COO-(PG-1), COO(C₁-C₆)-Alkyl oder CON-(PG-2)₂ substituiert sein kann;
und PG-1, PG-2 und PG-3 die oben beschriebene Bedeutung haben,
zu Verbindungen der allgemeinen Formel VII 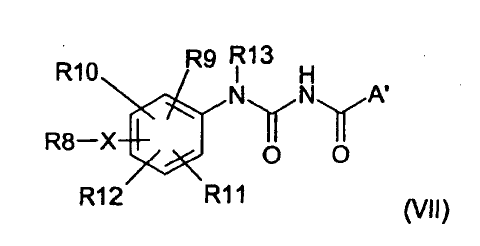 worin X, R8, R9, R10, R11, R12, R13 und A' die oben beschrieben Bedeutung haben; umsetzt und falls R1 in Verbindungen der allgemeinen Formel I kein Wasserstoffatom darstellt, diese weiterhin mit Verbindungen der allgemeinen Formel VIII
R14-LG (VIII)
worin LG die oben beschriebene Bedeutung hat und worin
R14 H, (C₁-C₆)-Alkyl, O-(C₁-C₆)-Alkyl, CO-(C₁-C₆)-Alkyl, COO-(C₁-C₆)-Alkyl, (C₁-C₆)-Alkylen-COO-(PG-1), (C₁-C₆)-Alkylen-COO-(C₁-C₆)-alkyl;
worin PG-1 die oben beschriebene Bedeutung hat;
unter Verwendung einer Base, wie zum Beispiel 1,8-Diazabicyclo[5.4.0]undec-7-en, so zu Verbindungen der allgemeinen Formel IX 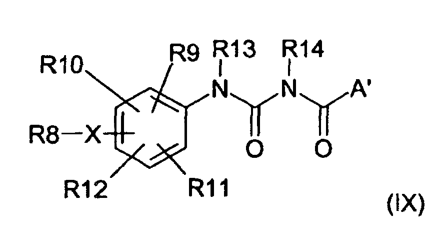 worin X, R8, R9, R10, R11, R12, R13, R14 und A' die oben beschrieben Bedeutung haben,
alkyliert
und gegebenenfalls die vorhandenen Schutzgruppen in den Resten R8, R9, R10, R11, R12, R13, R14, A' und B' abspaltet und gegebenenfalls die so erhaltenen Verbindungen der Formel I durch Zugabe eines Äquivalentes der entsprechenden Säure oder Base in deren Salze überführt.

## Claims

1. A compound of the formula I 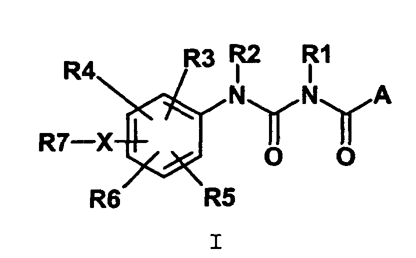 in which
A is phenyl, naphthyl, it being possible for the phenyl or naphthyl radical to be substituted up to three times by F, Cl, Br, OH, CF₃, NO₂, CN, OCF₃, O-(C₁-C₆)-alkyl, O-(C₁-C₆)-alkenyl, O-(C₁-C₆)-alkynyl, S-(C₁-C₆)-alkyl, S-(C₁-C₆)-alkenyl, S-(C₁-C₆)-alkynyl, SO-(C₁-C₆)-alkyl, SO₂-(C₁-C₆) -alkyl, SO₂-NH₂, (C₁-C₆)-alkyl, (C₁-C₆)-alkenyl, (C₁-C₆)-alkynyl, (C₃-C₇)-cycloalkyl, (C₃-C₇)-cycloalkyl-(C₁-C₄)-alkylene, (C₀-C₆)-alkylene-COOH, (C₀-C₆)-alkylene-COO(C₁-C₇)-alkyl, CONH₂, CONH(C₁-C₆)-alkyl, CON[(C₁-C₆)-alkyl]₂, CONH(C₃-C₆)-cycloalkyl, (C₀-C₆)-alkylene-NH₂, (C₀-C₆)-alkylene-NH(C₂-C₆)-alkyl, (C₀-C₆)-alkylene-N[(C₁-C₆)-alkyl]₂, NH-CO-(C₁-C₆)-alkyl, NH-CO-phenyl, NH-SO₂-phenyl, it being possible for the phenyl ring to be substituted up to twice by F, Cl, CN, OH, (C₁-C₆)-alkyl, O-(C₁-C₆)-alkyl, CF₃, OCF₃, COOH, COO(C₁-C₆)-alkyl or CONH₂;
R1, R2 are, independently of one another, H, (C₁-C₆)-alkyl, O-(C₁-C₆)-alkyl, CO-(C₁-C₆)-alkyl, COO-(C₁-C₆)-alkyl, (C₁-C₆)-alkylene-COOH, (C₁-C₆)-alkylene-COO-(C₁-C₆)-alkyl;
R3, R4, R5, R6 are, independently of one another, H, F, Cl, Br, OH, CF₃, NO₂, CN, OCF₃, O-(C₁-C₆)-alkyl, O-(C₁-C₆)-alkenyl, O-(C₁-C₆)-alkynyl , S-(C₁-C₆)-alkyl, S-(C₁-C₆)-alkenyl, S-(C₁-C₆)-alkynyl, SO-(C₁-C₆)-alkyl, SO₂-(C₁-C₆)-alkyl, SO₂-NH₂, (C₁-C₆)-alkyl, (C₁-C₆)-alkenyl, (C₁-C₆)-alkynyl, (C₃-C₇)-cycloalkyl, (C₃-C₇)-cycloalkyl-(C₁-C₄)-alkylene, COOH, COO(C₁-C₆)-alkyl, CONH₂, CONH(C₁-C₆)-alkyl, CON [(C₁-C₆)-alkyl]₂, CONH(C₃-C₇)-cycloalkyl, NH₂, NH(C₁-C₆)-alkyl, N[(C₁-C₆)-alkyl]₂, NH-CO-(C₁-C₆)-alkyl, NH-CO-phenyl, NH-SO₂-phenyl, it being possible for the phenyl ring to be substituted up to twice by F, Cl, CN, OH, (C₁-C₆)-alkyl, O-(C₁-C₆)-alkyl, CF₃, OCF₃, COOH, COO(C₁-C₆)-alkyl or CONH₂;
X is O, S;
R7 is (C₁-C₁₀)-alkylene-COOH, (C₆-C₁₀)-alkylene-COO-(C₁-C₆)-alkyl, (C₁-C₁₀)-alkylene-CONH₂, (C₁-C₁₀)-alkylene-CONH- (C₁-C₆)-alkyl, (C₁-C₁₀)-alkylene-CON- [(C₁-C₆)-alkyl]₂, (C₁-C₁₀)-alkylene-NH₂, (C₁-C₁₀)-alkylene-NH(C₁-C₆)-alkyl, (C₁-C₁₀)-alkylene-N [(C₁-C₆)-alkyl]₂, (C₁-C₁₀)-alkylene-B;
B is (C₃-C₇)-cycloalkyl, pyrrolyl, imidazolyl, thiazolyl, azetidinyl, thienyl, piperidinyl, pyrrolidinyl, morpholinyl, pyridyl-methyl or furyl, in which cycloalkyl, pyrrolyl, imidazolyl, thiazolyl, azetidinyl, thienyl, piperidinyl, pyrrolidinyl, morpholinyl, pyridyl or furyl may in each case be substituted up to twice by Cl, F, CN, CF₃, OCF₃, COOH, COO-(C₁-C₆)-alkyl, CONH₂, CONH-(C₁-C₆)-alkyl, CON- [(C₁-C₆)-alkyl]₂, (C₁-C₆) -alkyl, OH, O-(C₁-C₆)-alkyl;
and physiologically tolerated salts thereof,
excepting the compound of the formula 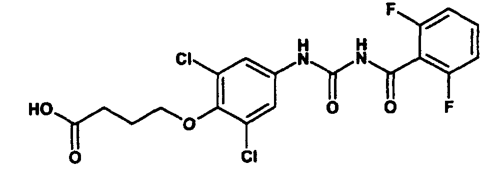 and compounds of the formula I in which the radicals mean at the same time
A phenyl;
X O;
R1 H;
R7 -(C₁-C₄)-alkyl-B;
B (C₃-C₇)-cycloalkyl, heteroaryl.

2. A compound of the formula I as claimed in claim 1 wherein
A is phenyl, it being possible for the phenyl radical to be substituted up to twice by F, Cl, Br, O-(C₁-C₆)-alkyl;
R1, R2 are, independently of one another, H, (C₁-C₆)-alkyl, CO-(C₁-C₆)-alkyl;
R3, R4, R5, R6 are, independently of one another, H, Cl, F, (C₁-C₆)-alkyl, O-(C₁-C₆)-alkyl, -COO-(C₁-C₆)-alkyl;
X is O;
R7 is (C₁-C₁₀)-alkylene-COOH, (C₆-C₁₀)-alkylene-COO- (C₁-C₆)-alkyl, (C₁-C₁₀)-alkylene-CONH₂;
and the physiologically tolerated salts thereof,
excepting the compound of the formula

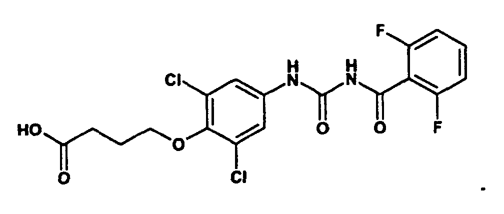

3. A pharmaceutical comprising one or more of the compounds as claimed in claim 1 or 2.

4. A pharmaceutical comprising one or more of the compounds as claimed in claim 1 or 2 and one or more blood glucose-lowering active ingredients.

5. The use of the compounds as claimed in claim 1 or 2 for producing a medicine.

6. The use of the compounds as claimed in claim 1 or 2 for producing a medicine for lowering blood glucose.

7. The use of the compounds as claimed in claim 1 or 2 for producing a medicine for treating type II diabetes.

8. The use of the compounds as claimed in claim 1 or 2 in combination with at least one other blood glucose-lowering active ingredient for producing a medicine.

9. A process for producing a pharmaceutical comprising one or more of the compounds as claimed in claim 1 or 2, which comprises mixing the active ingredient with a pharmaceutically acceptable carrier, and converting this mixture into a form suitable for administration.

10. The use of the compound of the formula I 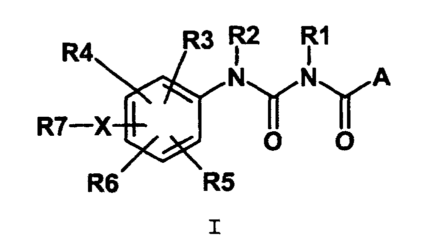 in which
A is phenyl, naphthyl, it being possible for the phenyl or naphthyl radical to be substituted up to three times by F, Cl, Br, OH, CF₃, NO₂, CN, OCF₃, O-(C₁-C₆)-alkyl, O-(C₁-C₆)-alkenyl, O-(C₁-C₆)-alkynyl, S-(C₁-C₆)-alkyl, S-(C₁-C₆)-alkenyl, S-(C₁-C₆)-alkynyl, SO-(C₁-C₆)-alkyl, SO₂-(C₁-C₆)-alkyl, SO₂-NH₂, (C₁-C₆)-alkyl, (C₁-C₆)-alkenyl, (C₁-C₆)-alkynyl, (C₃-C₇)-cycloalkyl, (C₃-C₇)-cycloalkyl-(C₁-C₄)-alkylene, (C₀-C₆)-alkylene-COOH, (C₀-C₆)-alkylene-COO(C₁-C₆)-alkyl, CONH₂, CONH(C₁-C₆)-alkyl, CON[(C₁-C₆)-alkyl]₂, CONH(C₃-C₇)-cycloalkyl, (C₀-C₆)-alkylene-NH₂, (C₀-C₆)-alkylene-NH(C₁-C₆)-alkyl, (C₀-C₆)-alkylene-N[(C₁-C₆)-alkyl]₂, NH-CO-(C₁-C₆)-alkyl, NH-CO-phenyl, NH-SO₂-phenyl, it being possible for the phenyl ring to be substituted up to twice by F, Cl, CN, OH, (C₁-C₆)-alkyl, O-(C₁-C₆)-alkyl, CF₃, OCF₃, COOH, COO(C₁-C₆)-alkyl or CONH₂;
R1, R2 are, independently of one another, H, (C₁-C₆)-alkyl, O-(C₁-C₆)-alkyl, CO-(C₁-C₆)-alkyl, COO-(C₁-C₆)-alkyl, (C₁-C₆)-alkylene-COOH, (C₁-C₆)-alkylene-COO- (C₁-C₆)-alkyl;
R3, R4, R5, R6 are, independently of one another, H, F, Cl, Br, OH, CF₃, NO₂, CN, OCF₃, O-(C₁-C₆)-alkyl, O- (C₁-C₆)-alkenyl, O- (C₁-C₆) -alkynyl, S-(C₁-C₆)-alkyl, S- (C₁-C₆)-alkenyl, S- (C₁-C₆)-alkynyl, SO-(C₁-C₆)-alkyl, SO₂-(C₁-C₆)-alkyl, SO₂-NH₂, (C₁-C₆)-alkyl, (C₁-C₆)-alkenyl, (C₁-C₆)-alkynyl, (C₃-C₇)-cycloalkyl, (C₃-C₇)-cycloalkyl-(C₁-C₄)-alkylene, COOH, COO(C₁-C₆)-alkyl, CONH₂, CONH(C₁-C₆)-alkyl, CON[(C₁-C₆)-alkyl]₂, CONH(C₃-C₇)-cycloalkyl, NH₂, NH(C₁-C₆)-alkyl, N[(C₁-C₆)-alkyl]₂, NH-CO-(C₁-C₆)-alkyl, NH-CO-phenyl, NH-SO₂-phenyl, it being possible for the phenyl ring to be substituted up to twice by F, Cl, CN, OH, (C₁-C₆)-alkyl, O-(C₁-C₆)-alkyl, CF₃, OCF₃, COOH, COO(C₁-C₆)-alkyl or CONH₂;
X is O, S;
R7 is (C₁-C₁₀)-alkylene-COOH, (C₁-C₁₀)-alkylene-COO-(C₁-C₆)-alkyl, (C₁-C₁₀)-alkylene-CONH₂, (C₁-C₁₀)-alkylene-CONH- (C₁-C₆)-alkyl, (C₁-C₁₀)-alkylene-CON- [ (C₁-C₆)-alkyl]₂, (C₁-C₁₀)-alkylene-NH₂, (C₁-C₁₀)-alkylene-NH(C₁-C₆)-alkyl, (C₁-C₁₀)-alkylene-N [(C₁-C₆)-alkyl]₂, (C₁-C₁₀)-alkylene-B;
B is (C₃-C₇)-cycloalkyl, phenyl, pyrrolyl, imidazolyl, thiazolyl, azetidinyl, thienyl, piperidinyl, pyrrolidinyl, morpholinyl, pyridyl or furyl, in which cycloalkyl, phenyl, pyrrolyl, imidazolyl, thiazolyl, azetidinyl, thienyl, piperidinyl, pyrrolidinyl, morpholinyl, pyridyl or furyl may in each case be substituted up to twice by Cl, F, CN, CF₃, OCF₃, COOH, COO-(C₁-C₆)-alkyl, CONH₂, CONH-(C₁-C₆)-alkyl, CON-[(C₁-C₆)-alkyl]₂, (C₁-C₆)-alkyl, OH, O-(C₁-C₆)-alkyl;
and physiologically tolerated salts thereof, for producing a medicine for lowering the blood glucose level.

11. A process for preparing the compounds as claimed in claim 1 or 2, wherein, in accordance with the following formula diagram 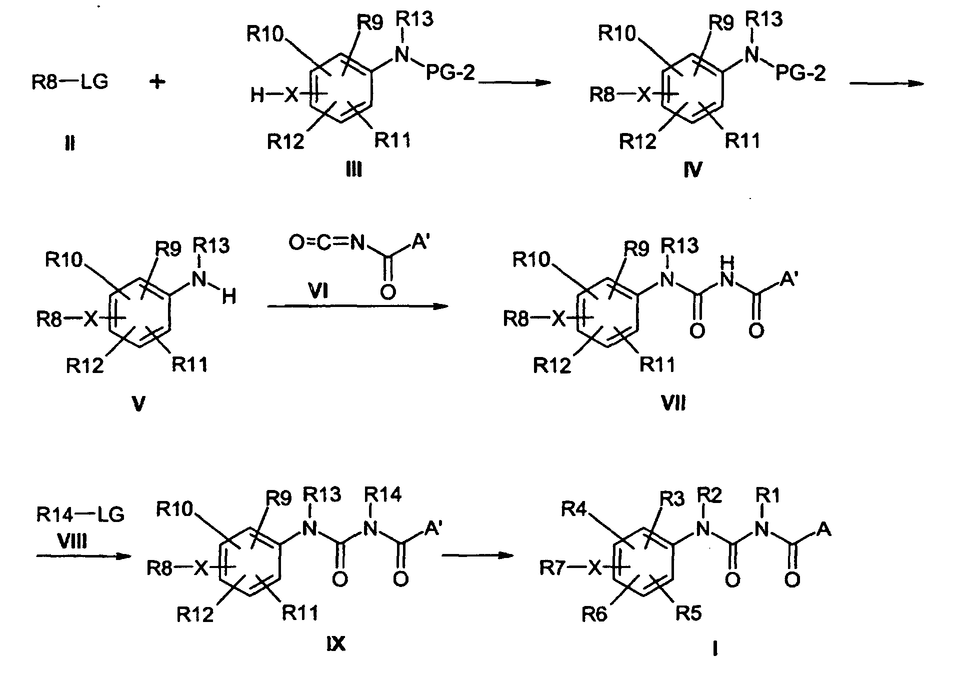 the compound of the formula II
R8-LG (II)
in which
R8 is (C₁-C₁₀)-alkylene-COO-(PG-1), (C₆-C₁₀)-alkylene-COO- (C₁-C₆)-alkyl, (C₁-C₁₀)-alkylene-CON-(PG-2)₂, (C₁-C₁₀)-alkylene-CONH-(C₁-C₆)-alkyl, (C₁-C₁₀)-alkylene-CON- [(C₁-C₆)-alkyl]₂, (C₁-C₁₀)-alkylene-N- (PG-2)₂, (C₁-C₁₀)-alkylene-NH (C₁-C₆)-alkyl , (C₁-C₁₀)-alkylene-N [(C₁-C₆)-alkyl]₂, (C₁-C₁₀)-alkylene-B'
PG-1 is a generally known protective group for esters, such as, for example, (C₁-C₆)-alkyl, benzyl or p-methoxybenzyl;
PG-2 is a generally known protective group for amino groups, such as, for example, (C₁-C₆)-alkylcarbonyl, (C₁-C₆)-alkyloxycarbonyl or (C₆-C₁₂)-aryl-(C₁-C₄)-alkyloxycarbonyl, which replaces either both hydrogens or only one hydrogen atom in the amino group;
B' is (C₃-C₇)-cycloalkyl, pyrrolyl, imidazolyl, thiazolyl, azetidinyl, thienyl, piperidinyl, pyrrolidinyl, morpholinyl, pyridylmethyl and furyl in which cycloalkyl, pyrrolyl, imidazolyl, thiazolyl, azetidinyl, thienyl, piperidinyl, pyrrolidinyl, morpholinyl, pyridyl and furyl may in each case be substituted up to twice by Cl, F, CN, CF₃, OCF₃, COO-(PG-1), COO-(C₁-C₆)-alkyl, CON-(PG-2)₂, CONH- (C₁-C₆)-alkyl, CON- [(C₁-C₆)-alkyl]₂, (C₁-C₆)-alkyl, O-(PG-3), O-(C₁-C₆)-alkyl;
PG-3 is a generally known protective group for alcohols, such as, for example, benzyl, allyl, tetrahydropyranyl or tetrahydrofuranyl;
LG is a generally known leaving group such as, for example, halogen, arylsulfonyloxy or alkylsulfonyloxy;
is alkylated with anilines of the formula III 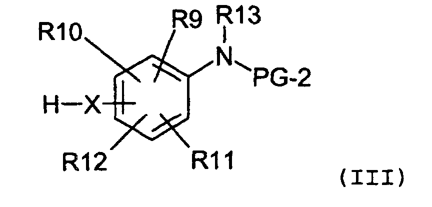 in which X and PG-2 have the meaning described above, and in which
R9, R10, R11, R12 are, independently of one another, H, F, Cl, Br, O-(PG-3), CF₃, NO₂, CN, OCF₃, O- (C₁-C₆)-alkyl, O- (C₁-C₆)-alkenyl, O-(C₁-C₆)-alkynyl, S-(C₁-C₆)-alkyl, S-(C₁-C₆)-alkenyl, S-(C₁-C₆)-alkynyl, SO-(C₁-C₆)-alkyl, SO₂-(C₁-C₆)-alkyl, SO₂-N-(PG-2)₂, (C₁-C₆)-alkyl, (C₁-C₆)-alkenyl, (C₁-C₆)-alkynyl, (C₃-C₇)-cycloalkyl, (C₃-C₇)-cycloalkyl-(C₁-C₄)-alkylene, COO-(PG-1), COO(C₁-C₆)-alkyl, CON-(PG-2)₂, CONH(C₁-C₆)-alkyl, CON[(C₁-C₆)-alkyl]₂, CONH(C₃-C₇)-cycloalkyl, N-(PG-2)₂, NH(C₁-C₆)-alkyl , N [(C₁-C₆)-alkyl]₂, NH-CO-(C₁-C₆)-alkyl, NH-CO-phenyl, NH-SO₂-phenyl, it being possible for the phenyl ring to be substituted up to twice by F, Cl, CN, O-(PG-3), (C₁-C₆)-alkyl, O-(C₁-C₆)-alkyl, CF₃, OCF₃, COO-(PG-1), COO(C₁-C₆)-alkyl or CON-(PG-2)₂;
R13 is H, (C₁-C₆)-alkyl, O-(C₁-C₆)-alkyl, CO-(C₁-C₆)-alkyl, COO-(C₁-C₆)-alkyl, (C₁-C₆)-alkylene-COO-(PG-1), (C₁-C₆)-alkylene-COO-(C₁-C₆)-alkyl;
in which PG-1, PG-2 and PG-3 have the meaning described above;
using a base such as, for example, potassium or cesium carbonate, in an organic solvent such as, for example, acetone or dimethylformamide,
to give compounds of the formula IV 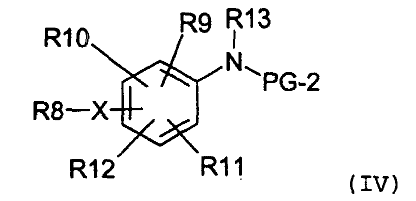 in which X, R8, R9, R10, R11, R12, R13 and PG-2 have the meaning described above,
and the latter are then converted, by selective elimination of the protective group PG-2, into compounds of the formula V 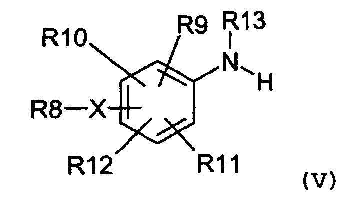 in which X, R8, R9, R10, R11, R12 and R13 have the meanings stated above;
and then the latter are reacted with isocyanates of the formula VI 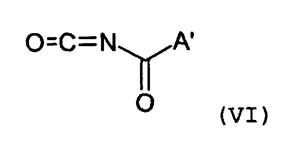 in which
A' is phenyl, naphthyl, it being possible for the phenyl or naphthyl radical to be substituted up to three times by F, Cl, Br, O-(PG-3), CF₃, NO₂, CN, OCF₃, O-(C₁-C₆)-alkyl, O-(C₁-C₆)-alkenyl, O-(C₁-C₆)-alkynyl, S-(C₁-C₆)-alkyl, S-(C₁-C₆)-alkenyl, S-(C₁-C₆)-alkynyl, SO-(C₁-C₆)-alkyl, SO₂-(C₁-C₆)-alkyl, SO₂-N-(PG-2)₂, (C₁-C₆)-alkyl, (C₁-C₆)-alkenyl, (C₁-C₆)-alkynyl, (C₃-C₇)-cycloalkyl, (C₃-C₇)-cycloalkyl- (C₁-C₄)-alkylene, (C₀-C₆)-alkylene-COO-(PG-1), (C₀-C₆)-alkylene-COO(C₁-C₆)-alkyl, CON-(PG-2)₂, CONH(C₁-C₆)-alkyl, CON[(C₁-C₆)-alkyl]₂, CONH(C₃-C₇)-cycloalkyl, (C₀-C₆)-alkylene-N-(PG-2)₂, (C₀-C₆)-alkylene-NH(C₁-C₆)-alkyl, (C₀-C₆)-alkylene-N [(C₁-C₆)-alkyl]₂, NH-CO- (C₁-C₆)-alkyl , NH-CO-phenyl, NH-SO₂-phenyl, it being possible for the phenyl ring to be substituted up to twice by F, Cl, CN, O-(PG-3), (C₁-C₆)-alkyl, O-(C₁-C₆)-alkyl, CF₃, OCF₃, COO-(PG-1), COO(C₁-C₆)-alkyl or CON-(PG-2)₂;
and PG-1, PG-2 and PG-3 have the meaning described above,
to give compounds of the formula VII 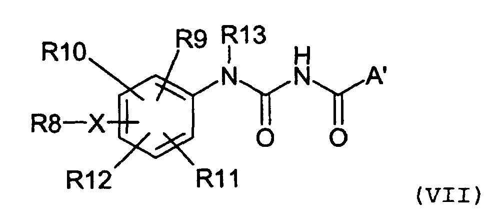 in which X, R8, R9, R10, R11, R12, R13 and A' have the meaning described above; and the latter are, if R1 in compounds of the formula I is not a hydrogen atom, further alkylated with compounds of the formula VIII
R14-LG (VIII)
in which LG has the meaning described above, and in which
R14 is H, (C₁-C₆)-alkyl, O-(C₁-C₆)-alkyl, CO-(C₁-C₆)-alkyl, COO-(C₁-C₆)-alkyl, (C₁-C₆)-alkylene-COO-(PG-1), (C₁-C₆)-alkylene-COO-(C₁-C₆)-alkyl;
in which PG-1 has the meaning described above; using a base such as, for example, 1,8-diazabicyclo[5.4.0]undec-7-ene to give compounds of the formula IX 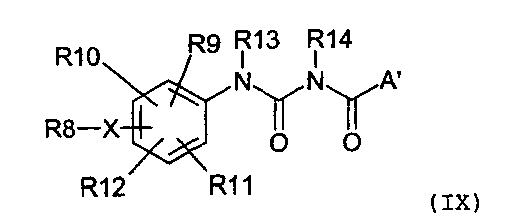 in which X, R8, R9, R10, R11, R12, R13, R14 and A' have the meaning described above,
and, where appropriate, the protective groups which are present in the radicals R8, R9, R10, R11, R12, R13, R14, A' and B' are eliminated and, where appropriate, the compounds of the formula I obtained in this way are converted into the salts thereof by adding one equivalent of the appropriate acid or base.

## Revendications

1. Composés de formule I 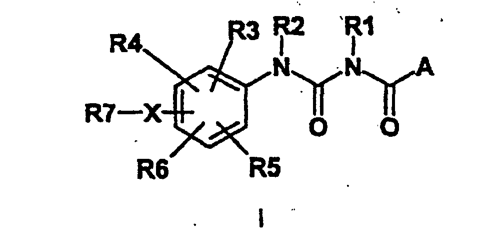 où représentent
A phényle, naphtyle, le reste phényle ou naphtyle pouvant être substitué jusqu'à trois fois par des substituants F, Cl, Br, OH, CF₃, NO₂, CN, OCF₃, O-alkyle en C₁-C₆, O-alcényle en C₁-C₆, O-alcynyle en C₁-C₆, S-alkyle en C₁-C₆, S-alcényle en C₁-C₆, S-alcynyle en C₁-C₆, SO-alkyle en C₁-C₆, SO₂-alkyle en C₁-C₆, SO₂-NH₂, alkyle en C₁-C₆, alcényle en C₁-C₆, alcynyle en C₁-C₆, cycloalkyle en C₃-C₇, (cycloalkyle en C₃-C₇)-alkylène en C₁-C₄, (alkylène en C₀-C₆)-COOH, (alkylène en C₀-C₆)-COO-alkyle en C₁-C₇, CONH₂, CONH-alkyle en C₁-C₆, CON(alkyle en C₁-C₆)₂, CONH-cycloalkyle en C₃-C₆, (alkylène en C₀-C₆)NH₂, (alkylène en C₀-C₆)NH-alkyle en C₂-C₆, (alkylène en C₀-C₆)N-(alkyle en C₁-C₆)₂, NH-CO-alkyle en C₁-C₆, NH-CO-phényle, NH-SO₂-phényle, le cycle phényle pouvant être substitué jusqu'à deux fois par des substituants F, Cl, CN, OH, alkyle en C₁-C₆, O-alkyle en C₁-C₆, CF₃, OCF₃, COOH, COO-alkyle en C₁-C₆ ou CONH₂ ;
R1, R2 indépendamment l'un de l'autre représentent un atome d'hydrogène, un groupe alkyle en C₁-C₆, O-alkyle en C₁-C₆, CO-alkyle en C₁-C₆, COO-alkyle en C₁-C₆, (alkylène en C₁-C₆)-COOH, (alkylène en C₁-C₆)-COO-(alkyle en C₁-C₆) ;
R3, R4, R5, R6 indépendamment l'un de l'autre représentent un atome d'hydrogène, F, Cl, Br, OH, CF₃, NO₂, CN, OCF₃, O-alkyle en C₁-C₆, O-alcényle en C₁-C₆, O-alcynyle en C₁-C₆, S-alkyle en C₁-C₆, S-alcényle en C₁-C₆, S-alcynyle en C₁-C₆, SO-alkyle en C₁-C₆, SO₂-alkyle en C₁-C₆, SO₂-NH₂, alkyle en C₁-C₆, alcényle en C₁-C₆, alcynyle en C₁-C₆, cycloalkyle en C₃-C₇, (cycloalkyle en C₃-C₇)-alkylène en C₁-C₄, COOH, COO-alkyle en C₁-C₆, CONH₂, CONH-alkyle en C₁-C₆, CON(alkyle en C₁-C₆)₂, CONH-cycloalkyle en C₃-C₇, NH₂, NH-alkyle en C₁-C₆, N(alkyle en C₁-C₆)₂, NH-CO-alkyle en C₁-C₆, NH-CO-phényle, NH-SO₂-phényle, le cycle phényle pouvant être substitué jusqu'à deux fois par des substituants F, Cl, CN, OH, alkyle en C₁-C₆, O-alkyle en C₁-C₆, CF₃, OCF₃, COOH, COO-alkyle en C₁-C₆ ou CONH₂ ;
X représente un atome de O, S ;
R7 représente un groupe (alkylène en C₁-C₁₀)-COOH, (alkylène en C₆-C₁₀)-COO-(alkyle en C₁-C₆), (alkylène en C₁-C₁₀)-CONH₂, (alkylène en C₁-C₁₀)-CONH-alkyle en C₁-C₆, (alkylène en C₁-C₁₀)-CON-(alkyle en C₁-C₆)₂, (alkylène en C₁-C₁₀)-NH₂, (alkylène en C₁-C₁₀)-NH-alkyle en C₁-C₆, (alkylène en C₁-C₁₀)-N (alkyle en C₁-C₆)₂, (alkylène en C₁-C₁₀)-B ;
B représente un groupe cycloalkyle en C₃-C₇, pyrrolyle, imidazolyle, thiazolyle, azétidinyle, thiényle, pipéridinyle, pyrrolidinyle, morpholinyle, pyridyl-méthyl ou furyle, les groupes cycloalkyle, pyrrolyle, imidazolyle, thiazolyle, azétidinyle, thiényle, pipéridinyle, pyrrolidinyle, morpholinyle, pyridyle ou furyle chacun pouvant être substitué jusqu'à deux fois par des substituants Cl, F, CN, CF₃, OCF₃, COOH, COO-alkyle en C₁-C₆, CONH₂, CONH-alkyle en C₁-C₆, CON-(alkyle en C₁-C₆)₂, alkyle en C₁-C₆, OH, O-alkyle en C₁-C₆ ;
ainsi que leurs sels physiologiquement tolérés, le composé de formule 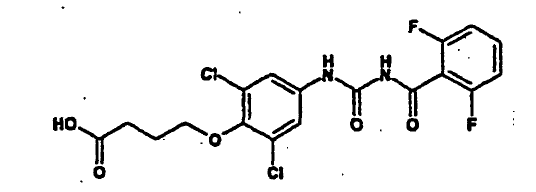 ainsi que des composés de formule I, où les restes représentent en même temps
A un groupe phényle ;
X un atome O ;
R1 un atome d'hydrogène ;
R7 un groupe (alkyle en C₁-C₄)-B;
B représente un groupe cycloalkyle C₃-C₇, hétéroaryle ;
étant exclus.

2. Composés de formule I selon la revendication 1, **caractérisés en ce que**
A représente un reste phényle, le reste phényle pouvant être substitué jusqu'à deux fois par des substituants F, Cl, Br, O-alkyle en C₁-C₆ ;
R1,R2 indépendamment l'un de l'autre représentent un atome d'hydrogène, un groupe alkyle en C₁-C₆, CO-alkyle en C₁-C₆ ;
R3, R4, R5, R6 indépendamment l'un de l'autre représentent un atome d'hydrogène, Cl, F, alkyle en C₁-C₆, O-alkyle en C₁-C₆, COO-alkyle en C₁-C₆ ;
X représente un atome de O ;
R7 représente un groupe (alkylène en C₁-C₁₀)-COOH, (alkylène en C₆-C₁₀)-COO- (alkyle en C₁-C₆), (alkylène en C₁-C₁₀)-CONH₂ ;
ainsi que leurs sels physiologiquement tolérés, le composé de formule 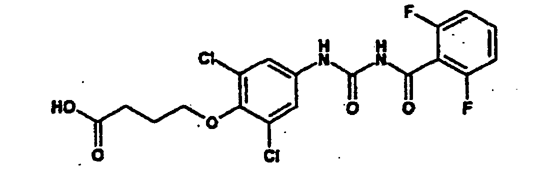 étant exclu.

3. Médicament renfermant un ou plusieurs des composés selon la revendication 1 ou 2.

4. Médicament renfermant un ou plusieurs des composés selon la revendication 1 ou 2 et un ou plusieurs principes actifs réduisant le niveau de glucose dans le sang.

5. Utilisation des composés selon la revendication 1 ou 2 pour la préparation d'un médicament.

6. Utilisation des composés selon la revendication 1 ou 2 pour la préparation d'un médicament réduisant le niveau de glucose dans le sang.

7. Utilisation des composés selon la revendication 1 ou 2 pour la préparation d'un médicament pour le traitement du diabète type 2.

8. Utilisation des composés selon la revendication 1 ou 2 en combinaison avec au moins un autre principe actif réduisant le niveau de glucose dans le sang pour la préparation d'un médicament.

9. Procédé pour la préparation d'un médicament renfermant un ou plusieurs des composés selon la revendication 1 ou 2, **caractérisé en ce qu'**on mélange le principe actif avec un véhicule approprié en pharmacie et ce mélange est mis en forme d'administration appropriée.

10. Utilisation du composé de formule I 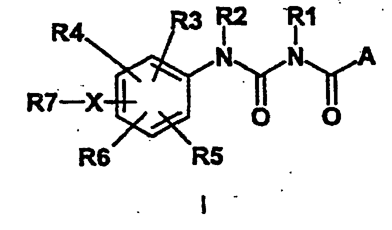 où
A représente un groupe phényle, naphtyle, le reste phényle ou naphtyle pouvant être substitué jusqu'à trois fois par des substituants F, Cl, Br, OH, CF₃, NO₂, CN, OCF₃, O-alkyle en C₁-C₆, O-alcényle en C₁-C₆, O-alcynyle en C₁-C₆, S-alkyle en C₁-C₆, S-alcényle en C₁-C₆, S-alcynyle en C₁-C₆, SO-alkyle en C₁-C₆, SO₂-alkyle en C₁-C₆, SO₂-NH₂, alkyle en C₁-C₆, alcényle en C₁-C₆, alcynyle en C₁-C₆, cycloalkyle en C₃-C₇, (cycloalkyle en C₃-C₇)-alkylène en C₁-C₄, (alkylène en C₀-C₆)-COOH, (alkylène en C₀-C₆)COO-alkyle en C₂-C₆, CONH₂, CONH-alkyle en C₁-C₆, CON(alkyle en C₁-C₆)₂, CONH-cycloalkyle en C₃-C₇, (alkylène en C₀-C₆)NH₂, (alkylène en C₀-C₆)-NH-alkyle en C₁-C₆, (alkylène en C₀-C₆)-N-(alkyle en C₁-C₆)₂, NH-CO-alkyle en C₁-C₆, NH-CO-phényle, NH-SO₂-phényle, le cycle phényle pouvant être substitué jusqu'à deux fois par des substituants F, Cl, CN, OH, alkyle en C₁-C₆, O-alkyle en C₁-C₆, CF₃, OCF₃, COOH, COO-alkyle en C₁-C₆ ou CONH₂ ;
R1, R2 indépendamment l'un de l'autre représentent un atome d'hydrogène, un groupe alkyle en C₁-C₆, O-alkyle en C₁-C₆, CO-alkyle en C₁-C₆, COO-alkyle en C₁-C₆, (alkylène en C₁-C₆)-COOH, (alkylène en C₁-C₆)-COO-alkyle en C₁-C₆,
R3, R4, R5, R6 indépendamment les uns des autres représentent un atome d'hydrogène, F, Cl, Br, OH, CF₃, NO₂, CN, OCF₃, O-alkyle en C₁-C₆, O-alcényle en C₁-C₆, O-alcynyle en C₁-C₆, S-alkyle en C₁-C₆, S-alcényle en C₁-C₆, S-alcynyle en C₁-C₆, SO-alkyle en C₁-C₆, SO₂-alkyle en C₁-C₆, SO₂-NH₂, alkyle en C₁-C₆, alcényle en C₁-C₆, alcynyle en C₁-C₆, cycloalkyle en C₃-C₇, (cycloalkyl en C₃-C₇)-alkylène en C₁-C₄, COOH, COO-alkyle en C₁-C₆, CONH₂, CONH-alkyle en C₁-C₆, CON(alkyle en C₁-C₆)₂, CONH-cycloalkyle en C₃-C₇, NH₂, NH-alkyle en C₁-C₆, N(alkyle en C₁-C₆)₂, NH-CO-alkyle en C₁-C₆, NH-CO-phényle, NH-SO₂-phényle, le cycle phényle pouvant être substitué jusqu'à deux fois par des substituants F, Cl, CN, OH, alkyle en C₁-C₆, O-alkyle en C₁-C₆, CF₃, OCF₃, COOH, COO-alkyle en C₁-C₆ ou CONH₂ ;
X représente un atome de 0, S ;
R7 représente un groupe (alkylène en C₁-C₁₀)-COOH, (alkylène en C₁-C₁₀)-COO- (alkyle en C₁-C₆), (alkylène en C₁-C₁₀)-CONH₂, (alkylène en C₁-C₁₀)-CONH-alkyle en C₁-C₆, (alkylène en C₁-C₁₀)-CON-(alkyle en C₁-C₆)₂, (alkylène en C₁-C₁₀)-NH₂, (alkylène en C₁-C₁₀)-NH-alkyle en C₁-C₆, (alkylène en C₁-C₁₀)-N-(alkyle en C₁-C₆)₂, (alkylène en C₁-C₁₀)-B ;
B représente un groupe cycloalkyle en C₃-C₇, phényle, pyrrolyle, imidazolyle, thiazolyle, azétidinyle, thiényle, pipéridinyle, pyrrolidinyle, morpholinyle, pyridyle ou furyle, où les groupes cycloalkyle, phényle, pyrrolyle, imidazolyle, thiazolyle, azétidinyle, thiényle, pipéridinyle, pyrrolidinyle, morpholinyle, pyridyle ou furyle chacun pouvant être substitué jusqu'à deux fois par des substituants Cl, F, CN, CF₃, OCF₃, COOH, COO-alkyle en C₁-C₆, CONH₂ CONH-alkyle en C₁-C₆, CON-(alkyle en C₁-C₆)₂, alkyle en C₁-C₆, OH, O-alkyle en C₁-C₆ ;
ainsi que leurs sels physiologiquement tolérés, pour la préparation d'un médicament pour réduire le niveau de glucose dans le sang.

11. Procédé pour la préparation des composés selon la revendication 1 ou 2, **caractérisé en ce que**, selon le schéma suivant 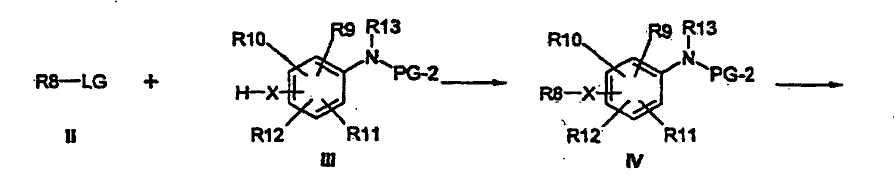 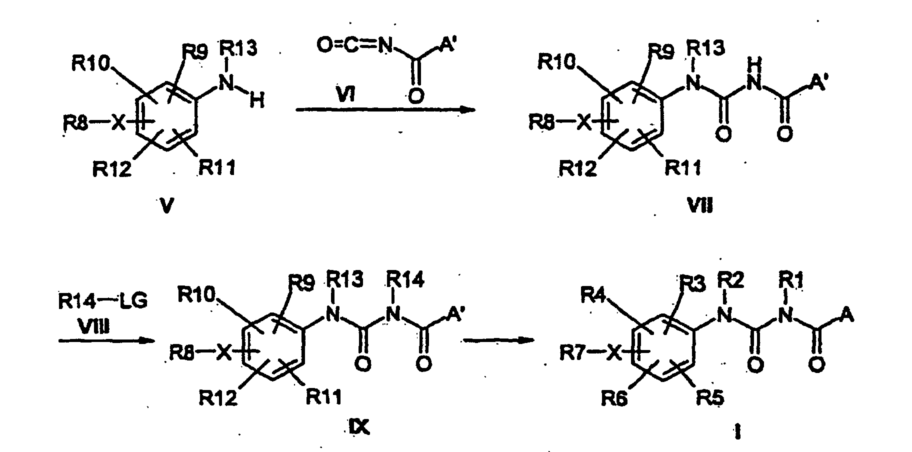 on alkyle le composé de formule générale II
**R8-LG (II)**
où
R8 représente un groupe (alkylène en C₁-C₁₀)-COO-(PG-1), (alkylène en C₆-C₁₀)-COO-alkyle en C₁-C₆, (alkylène en C₁-C₁₀)-CON- (PG-2) ₂, (alkylène en C₁-C₁₀)-CONH-alkyle en C₁-C₆, (alkylène en C₁-C₁₀)-CON-(alkyle en C₁-C₆)₂, (alkylène en C₁-C₁₀)-N-(PG-2)₂, (alkylène en C₁-C₁₀)-NH-alkyle en C₁-C₆, (alkylène en C₁-C₁₀) -N- (alkyle en C₁-C₆)₂, (alkylène en C₁-C₁₀)-B',
PG-1 est un groupe protecteur d'esters généralement connu, comme par exemple alkyle en C₁-C₆, benzyle ou p-méthoxybenzyle ;
PG-2 est un groupe protecteur de groupes amino généralement connu, comme par exemple (alkyl en C₁-C₆)-carbonyle, (alkyloxy en C₁-C₆)carbonyle ou (aryl en C₆-C₁₂)-alkyloxycarbonyle en C₁-C₄, qui remplace soit les deux atomes d'hydrogène soit seulement un atome d'hydrogène du groupe amino ;
B' représente un groupe cycloalkyle en C₃-C₇, pyrrolyle, imidazolyle, thiazolyle, azétidinyle, thiényle, pipéridinyle, pyrrolidinyle, morpholinyle, pyridyl-méthyle et furyle, où les groupes cycloalkyle, pyrrolyle, imidazolyle, thiazolyle, azétidinyle, thiényle, pipéridinyle, pyrrolidinyle, morpholinyle, pyridyle et furyle chacun pouvant être substitué jusqu'à deux fois par des substituants Cl, F, CN, CF₃, OCF₃, COO-(PG-1), COO-alkyle en C₁-C₆, CON-(PG-2)₂, CONH-alkyle en C₁-C₆, CON-(alkyle en C₁-C₆)₂, alkyle en C₁-C₆, O-(PG-3), O-alkyle en C₁-C₆ ;
PG-3 est un groupe protecteur d'alcools généralement connu, comme par exemple benzyle, allyle, tétrahydropyranyle ou tétrahydrofuranyle ;
LG est un groupe éliminable, comme par exemple halogène, arylsulfonyloxy ou alkylsulfonyloxy ;
par des anilines de formule générale III 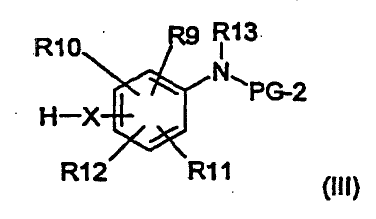 où X et PG-2 possèdent les significations décrites ci-dessus et où
R9, R10, R11, R12 indépendamment l'un de l'autre représentent un atome d'hydrogène, F, Cl, Br, O-(PG-3), CF₃, NO₂, CN, OCF₃, O-alkyle en C₁-C₆, O-alcényle en C₁-C₆, O-alcynyle en C₁-C₆, S-alkyle en C₁-C₆, S-alcényle en C₁-C₆, S-alcynyle en C₁-C₆, SO-alkyle en C₁-C₆, SO₂-alkyle en C₁-C₆, SO₂-N-(PG-2)₂, alkyle en C₁-C₆, alcényle en C₁-C₆, alcynyle en C₁-C₆, cycloalkyle en C₃-C₇, (cycloalkyle en C₃-C₇)-alkylène en C₁-C₄, COO-(PG-1), COO-alkyle en C₁-C₆, CON-(PG-2)₂, CONH-alkyle en C₁-C₆, CON(alkyle en C₁-C₆)₂, CONH-cycloalkyle en C₃-C₇, N-(PG-2)₂, NH-alkyle en C₁-C₆, N (alkyle en C₁-C₆)₂, NH-CO-alkyle en C₁-C₆, NH-CO-phényle, NH-SO₂-phényle, le cycle phényle pouvant être substitué jusqu'à deux fois par des substituants F, Cl, CN, O-(PG-3), alkyle en C₁-C₆, O-alkyle en C₁-C₆, CF₃, OCF₃, COO-(PG-1), COO-alkyle en C₁-C₆ ou CON-(PG-2)₂ ;
R13 représente un atome d'hydrogène, un groupe alkyle en C₁-C₆, O-alkyle en C₁-C₆, CO-alkyle en C₁-C₆, COO-alkyle en C₁-C₆, (alkylène en C₁-C₆)-COO-(PG-1), (alkylène en C₁-C₆)-COO-alkyle en C₁-C₆ ;
où PG-1, PG-2 et PG-3 possèdent la signification décrite ci-dessus ;
en utilisant une base, comme par exemple le carbonate de potassium ou de césium, dans un solvant organique, comme par exemple l'acétone ou le diméthylformamide,
en composés de formule générale IV 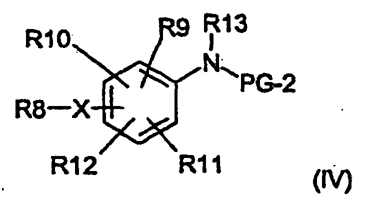 dans lesquels X, R8, R9, R10, R11, R12, R13 et PG-2 possèdent la signification décrite ci-dessus,
et ensuite on transforme ceux-ci par séparation sélective du groupe protecteur PG-2 en composés de formule générale V 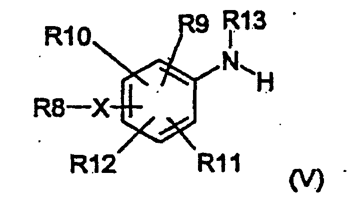 dans laquelle X, R8, R9, R10, R11, R12 et R13 possèdent la signification décrite ci-dessus ;
et ensuite on fait régir ceux-ci sur des isocyanates de formule générale VI 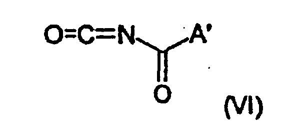 où
A' représente un groupe phényle, naphtyle, le reste phényle ou naphtyle pouvant être substitué jusqu'à trois fois par des substituants F, Cl, Br, O-(PG-3), CF₃, NO₂, CN, OCF₃, O-alkyle en C₁-C₆, O-alcényle en C₁-C₆, O-alcynyle en C₁-C₆, S-alkyle en C₁-C₆, S-alcényle en C₁-C₆, S-alcynyle en C₁-C₆, SO-alkyle en C₁-C₆, SO₂-alkyle en C₁-C₆, SO₂-N-(PG-2)₂, alkyle en C₁-C₆, alcényle en C₁-C₆, alcynyle en C₁-C₆, cycloalkyle en C₃-C₇, (cycloalkyle en C₃-C₇)-alkylène en C₁-C₄, (alkylène en C₀-C₆)-COO-(PG-1), (alkylène en C₀-C₆)COO-alkyle en C₁-C₆, CON-(PG-2)₂, CONH-alkyle en C₁-C₆, CON(alkyle en C₁-C₆)₂, CONH-cycloalkyle en C₃-C₇, (alkylène en C₀-C₆)N-(PG-2)₂, (alkylène en C₀-C₆)-NH-alkyle en C₁-C₆, (alkylène en C₀-C₆)-N-(alkyle en C₁-C₆)₂, NH-CO-alkyle en C₁-C₆, NH-CO-phényle, NH-SO₂-phényle, le cycle phényle pouvant être substitué jusqu'à deux fois par des substituants F, Cl, CN, O-(PG-3), alkyle en C₁-C₆, O-alkyle en C₁-C₆, CF₃, OCF₃, COO-(PG-1), COO-alkyle en C₁-C₆ ou CON-(PG-2)₂ ;
et PG-1, PG-2 et PG-3 possèdent la signification décrite ci-dessus,
pour obtenir les composés de formule générale VII 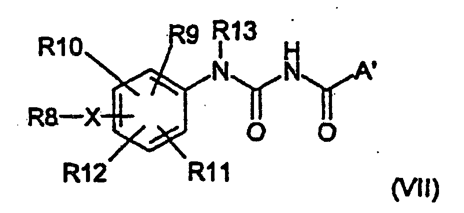 dans laquelle X, R8, R9, R10, R11, R12, R13 et A' possèdent la signification décrite ci-dessus ; et dans le cas où R1 dans les composés de formule générale I ne représente pas un atome d'hydrogène, on les fait réagir sur des composés de formule générale VIII
**R14-LG (VIII)**
où LG possède la signification donnée ci-dessus et où
R14 représente un atome d'hydrogène, un groupe alkyle en C₁-C₆, O-alkyle en C₁-C₆, CO-alkyle en C₁-C₆, COO-alkyle en C₁-C₆, (alkylène en C₁-C₆)-COO-
(PG-1), (alkylène en C₁-C₆)-COO-alkyle en C₁-C₆ ;
où PG-1 possède la signification donnée ci-dessus ; on alkyle en utilisant une base, comme par exemple le 1,8-diazabicyclo[5.4.0]undec-7-ène, pour obtenir les composés de formule générale IX 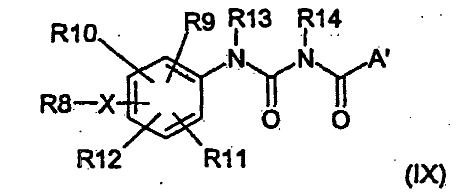 dans laquelle X, R8, R9, R10, R11, R12, R13, R14 et A' possèdent la signification décrite ci-dessus,
et éventuellement on sépare les groupes protecteurs présents dans les restes R8, R9, R10, R11, R12, R13, R14, A' et B' et éventuellement on transforme les composés de formule I ainsi obtenus en leurs sels par ajout d'un équivalent de l'acide ou de la base correspondante.
